# EUROPEAN PATENT APPLICATION

(11) **EP 2 684 958 A1**
(43) Date of publication of application: **15.01.2014**
(21) Application number: 13174782.6
(22) Date of filing: 05.04.2007
(51) Int. Cl.: C12N 15/52, C12N 15/12, C12N 5/12

(54) **Plant superoxide dismutase expression resistant to micro-RNA regulation**

(30) Priority: 19.07.2006 US 832038 P
(62) Divisional of application: 07774950.5
(71) Applicant: The Regents of The University of California, Oakland, CA 94607-5200 (US)
(72) Inventor: Sunkar, Ramanjulu, Riverside, CA92521 (US); Kapoor, Avnish, Riverside, CA92521 (US); Zhu, Jian-Kang, Riverside, CA92521 (US)
(74) Representative: Woolley, Lindsey Claire

(57) **Abstract**

This invention provides variant sequences to miR398 targets in copper/zinc superoxide dismutase (CSD) genes resulting in resistance to down regulation of the CSD by the miR398.

## Description

### CROSS-REFERENCE TO RELATED APPLICATIONS

This application claims priority to and benefit of a prior U.S. Provisional Application number 60/832,038, Plant Superoxide Dismutase Expression Resistant to Micro-RNA Regulation, by Ramanjulu Sunkar, et al., filed July 19, 2006. The full disclosure of the prior application is incorporated herein by reference.

### FIELD OF THE INVENTION

The present invention is in the field of micro-RNA regulation of plant proteins. In particular, the invention relates to regulation of micro-RNA in plants under oxidative stress and mutant superoxide dismutase mRNAs having reduced sensitivity to posttranscritional regulation by micro-RNAs.

### BACKGROUND OF THE INVENTION

MicroRNAs (miRNAs) are a class of regulatory RNAs of -21 nucleotides (nt) that post-transcriptionally regulate gene expression by directing mRNA cleavage or by translational inhibition. Increasing evidence points to a potential role of miRNAs in diverse physiological processes.

Regulation of gene expression at the transcriptional level is known to determine the developmental progression and physiological status in plants and animals. With the discovery of microRNAs (miRNAs) and small interfering RNAs, the importance of posttranscriptional gene regulation is also widely recognized now. miRNAs are ∼21-nt noncoding RNAs and are processed from hairpin precursors by the Dicer family of enzymes (Carrington and Ambros, 2003; Bartel, 2004; Baulcombe, 2004; He and Hannon, 2004). They repress gene expression by guiding effector complexes (miRNPs or RISC) to complementary sites on mRNAs (Bartel, 2004). Because of the extensive sequence complementarity between plant miRNAs and their target mRNAs, RISC recruitment in plants typically leads to target mRNA cleavage (Carrington and Ambros, 2003; Bartel, 2004; Schwab et al., 2005). Animal miRNAs are only partially complementary to their targets and thus typically repress expression by blocking translation initiation (Ambros, 2004; Bartel, 2004), although mRNA cleavage might also occur (Bagga et al., 2005; Pillai et al., 2005).

The involvement of plant miRNAs in various developmental processes such as phase transitions, flowering, and leaf and root development has been demonstrated (Aukerman and Sakai, 2003; Palatnik et al., 2003; Chen, 2004; Mallory et al., 2004; 2005; Vaucheret et al., 2004; Baker et al., 2005; Guo et al., 2005). Environmental stresses induce the expression of many genes. Increasing evidence also points to the potential role of miRNAs in various physiological processes. For example, miR395 and miR399 were recently shown to be induced by sulfate and phosphate deprivation, respectively, and the induction is important for the down-regulation of certain genes under nutrient deficiency stress (Jones-Rhoades and Bartel, 2004; Fujii et al., 2005; Chiou et al., 2006).

Accumulation of reactive oxygen species (ROS) as a result of various environmental stresses is a major cause of loss of crop productivity (Allen et al., 1997; Apel and Hirt, 2004; Mittler, 2004; Foyer and Noctor, 2005; Bartel and Sunkar, 2005). ROS affect many cellular functions by damaging nucleic acids, oxidizing proteins and causing lipid peroxidation (Foyer et al., 1994). Stress-induced ROS accumulation is counteracted by intrinsic antioxidant systems in plants that include a variety of enzymatic scavengers such as superoxide dismutase, ascorbate peroxidase, glutathione peroxidase, glutathione-S-transferase and catalase. In addition, nonenzymatic low-molecular-mass molecules such as ascorbate, tocopherol, carotenoids and glutathione may also be important (Mittler, 2002; Mittler et al., 2004). Plant stress tolerance may, therefore, be improved by the enhancement of in vivo levels of antioxidant enzymes (Mittler 2002).

Superoxide dismutases (SODs) constitute the first line of defense against highly toxic superoxide radicals by rapidly converting superoxide to hydrogen peroxide (H2O2) and molecular oxygen (Fridovich, 1995). On the basis of the metal co-factor used, SODs are classified into 3 groups: iron SOD (Fe-SOD), manganese SOD (Mn-SOD), and copper-zinc SOD (Cu-Zn SOD), which are localized in different cellular compartments (Mittler 2002). Overexpression of a Cu/Zn-SOD (a cytosolic SOD from Pea) in transgenic tobacco plants increased ozone tolerance (Pitcher and Zalinskas, 1996); Mn-SOD overproducing plants showed improved tolerance against freezing, water deficit, winter survival (McKersie et al., 1993; 1996; 1999) and methyl viologen-induced oxidative stress (Bowler et al., 1991; Slooten et al., 1995). Overexpression of Fe-SOD in transgenic plants also led to increased tolerance against methyl viologen (Van Camp et al., 1996; Van Breusegem et al., 1999) and winter survival (McKersie et al., 2000). Overexpression of a chloroplastic Cu/Zn-SOD from pea (ortholog of Arabidopsis CSD2) in transgenic tobacco plants resulted in increased tolerance against high light and low temperature stresses (Sen Gupta et al., 1993a; 1993b).

The recent discovery that miR398 targets CSD1 and CSD2 genes (Sunkar and Zhu, 2004; Jones-Rhoades and Bartel, 2004; Bonnet et al., 2004) has suggested a direct connection between the miRNA pathway and CSD1 and CSD2 regulation. miR398 and its target sites on CSD1 and CSD2 mRNA are conserved in dicotyledonous and monocotyledonous plants (Bonnet et al., 2004; Jones-Rhoades and Bartel, 2004; Sunkar and Zhu, 2004; Lu et al., 2005; Sunkar et al., 2005), but the potential functional consequences of miR398-guided CSD1 and CSD2 regulation has not been previously explored.

In view of the above, a need exists for a better understanding of how miRNAs are regulated. It would be desirable to have a way to assay systems for monitoring interactions between miRNAs and superoxide dismutase expression. The present invention provides these and other features that will be apparent upon review of the following.

### SUMMARY OF THE INVENTION

Resistance of plants to oxidative stress can be enhanced by increasing the activity of certain superoxide dismutase (SOD) enzyme activities within the plant's cells. However, increasing the copy number of the superoxide dismutase gene may not be effective in increasing the activity because expression can be post-transcriptionally down regulated, e.g., by micro-RNA associated complexes that repress translation or degrade targeted mRNA. The present invention overcomes this problem by providing superoxide dismutase genes encoding mRNAs resistant to the micro-RNA targeting.

A DNA polynucleotide sequence for a copper /zinc superoxide dismutase (CSD) can include sequences for micro-RNA targeting. For example, a target for micro-RNA 398 (miR398) in plant CSD1 can include the DNA sequence A AGG GGT TTC CTG AGA TCA CA (SEQ ID NO: 1). The miR398 target sequence in plant CSD2 can include the sequence T GCG GGT GAC CTG GGA AAC A (SEQ ID NO: 2). CSD genes with variants of the target sequences can encode mRNAs significantly resistant to regulation by miR398. Such CSD genes with target sequence variants can continue to encode active enzyme sequences while presenting a less effective target for the miR398. For example, the variants of SEQ ID NO: 1 can continue to encode the peptide sequence R G F L R S (SEQ ID NO: 3) or a conservative variation thereof, while providing mRNAs presenting a significantly less effective target for post transcriptional regulation by miR398 complexes. The CSD1 target sequence SEQ ID NO: 1 is actually located in the 5'UTR (untranslated region) of the CSD gene, so in many cases we find it does not matter if amino acid encoding is retained. Therefore, with regard to the CSD1 target sequence, optional embodiments of the invention routinely include mismatches in the first and/or second codon nucleotide (more than just "wobble" nucleotides) that would change encoding to different amino acids, non-conservative variations, or any amino acid (but, typically not to encode a start or stop codon). Alternately, variants of SEQ ID NO: 2 can be designed to continue to encode the peptide sequence H A G D L G N (SEQ ID NO: 4) or a conservative variation thereof, while providing mRNAs presenting a significantly less effective target for post transcriptional regulation by miR398 complexes. The less effective targets can result in significantly reduced miR398 post-transcriptional regulation, e.g., 5%, 10%, 25%, 50% 75%, 90%, 95%, 99%, or more, reduction in degradation or reduction in translation repression, as compared to regulation of the wild type CSD mRNAs. In preferred embodiments of the invention, the variant CSD sequences are present in plant cells.

In more preferred embodiments, polynucleotides of the invention is a variant of CSD sequence SEQ ID No: 1, such as A AGN GGN TTN CTN AGN TCN CA (SEQ ID NO: 26), AGN GGN TNN CTN AGN TCN CA (SEQ ID NO: 29) or a variant of CSD SEQ ID NO: 2, such as N GCN GGN GAN TTN GGN AAN A (SEQ ID NO: 27), or CAN GCN GGN GAN NTN GGN AAN A (SEQ ID NO: 30) wherein N is any nucleotide, and at least one of the Ns represents an nucleotide different from the corresponding nucleotide of SEQ ID NO: 1 or SEQ ID NO: 2. In more preferred embodiments, 2, 3, 4, 5, 6 or more for the nucleotides is changed in the variant as compared to the original sequence.

In a most preferred embodiment, the variant CSD sequence encodes a peptide that retains full superoxide dismutase enzymatic activity. For example, the variants can include sequence changes that do not change the amino acid encoded by the associated triplet codon, thus retaining the original (e.g., wild type) amino acid sequence of the enzyme. For example, where an original codon is AGG, encoding arginine (R), a variant can have the variant codon AGA - still encoding arginine in the translated peptide but providing a less attractive target for the miR398 regulatory complex.

In certain embodiments of the invention, at least one nucleotide base of all or most of the codons in the target sequence are different from the corresponding bases in the original wild type target sequence. In many cases the difference between wild type codons and variant codons will not result in the sequence encoding a different amino acid, e.g., due to the degeneracy of the genetic code. These different nucleic acid sequences encoding the same amino acid sequences can be considered conservative variations of each other. For example, the wild type CSD2 target sequence T GCG GGT GAC CTG GGA AAC A (SEQ ID NO: 2) can encode the amino acid sequence H A G D L G N (SEQ ID NO: 4), while the variant sequence CAT GCC GGA GAT TTA GGC AAT A (SEQ ID NO: 5) with 7 base changes located in 6 of the codons still encodes the amino acid sequence H A G D L G N (SEQ ED NO: 4). Optionally, the target sequences of the invention include differences between wild type codons and variant codons will result in the sequence encoding a different amino acid that is a conservative variation of the original amino acid, e.g., wherein the original wild type and variant amino acid have similar steric, hydrophobic, and/or charge properties, so that the enzyme activity is not substantially reduced.

The present invention includes methods of reducing miR398 post transcriptional regulation of CSD expression by introducing one or more by introducing one or more mismatching nucleotides into the miR398 target sequence of the CSD gene. In preferred embodiments, 3 to 7 mismatching nucleotides are introduced into the target sequence (e.g., beyond those mismatches already present between miR398 and the wild type CSD target sequence). For example, the target sequence with mismatched nucleotides can be A AGN GGN TNN CTN AGN TCN CA (SEQ ID NO: 26), or CAN GCN GGN GAN NTN GGN AAN A (SEQ ID NO: 30); where N is any nucleotide, and at least one of the Ns represents an nucleotide different from the corresponding nucleotide of the wild type sequence SEQ ID NO 1 or different from SEQ ID NO 5 sequence which encodes a wild type amino acid sequence of CSD2, respectively. The methods of the invention also provide for introduction into a plant of CSD genes having additional mismatching nucleotides in the miR398 target sequence as compared to the wild type target sequences.

### DEFINITIONS

Unless otherwise defined herein or below in the remainder of the specification, all technical and scientific terms used herein have meanings commonly understood by those of ordinary skill in the art to which the present invention belongs.

Before describing the present invention in detail, it is to be understood that this invention is not limited to particular assays or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a component" can include a combination of two or more components; reference to "a cell" can include mixtures of two or more cells, and the like.

Although many methods and materials similar, modified, or equivalent to those described herein can be used in the practice of the present invention without undue experimentation, the preferred materials and methods are described herein. In describing and claiming the present invention, the following terminology will be used in accordance with the definitions set out below.

The term "nucleotide", as used herein, refers to nucleotide bases which are incorporated into the polynucleotide sequences of the invention. With regard to DNA sequences - A, T, G, C (deoxyadenylate, thymidylate, deoxyguanylate, deoxycytidylate); and, with regard to RNA - A, U, G, C (adenylate, uridylate, guanylate, cytidylate). Nucleotides of the sequences can also be unnatural nucleotides or nucleotide analogs, such as, e.g., azo-purines, deaza-adenosine, oxo-pyridines, and the like.

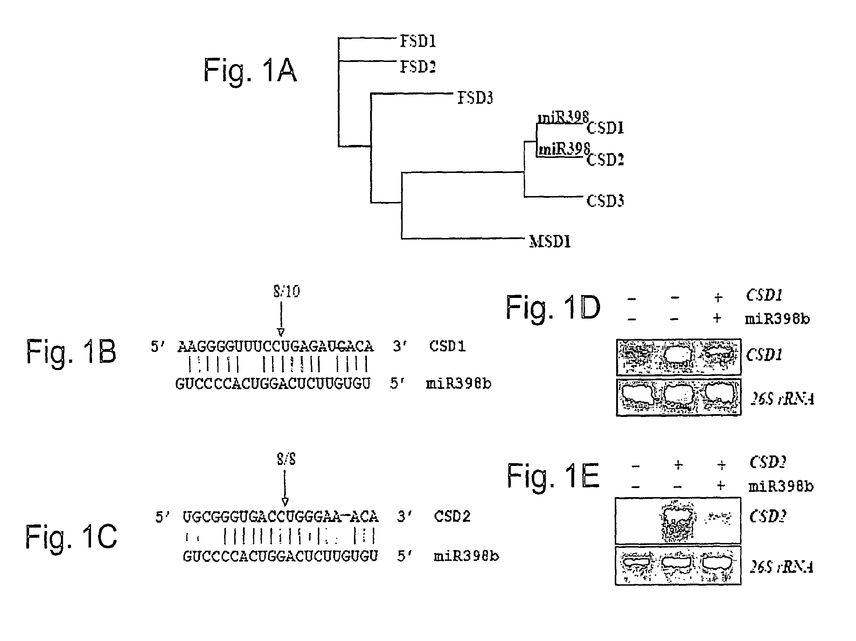

The term "mismatched" refers to a lack of base pair complementarity between, e.g., a miRNA and its mRNA target sequence. For example, when a miRNA and mRNA target sequence are aligned antiparallel, many of the bases are paired off for appropriate hydrogen bonding interactions between A:U or G:C. However, other base pairs in this alignment are not complimentary (e.g., A:G, A:C, or C:U) and are considered mismatched (see, for example, the matches and mismatches between miR398 and CSD target sequences in Figure 1B and 1C.

The term "variant sequence", as used herein, refers to a variant of a miRNA target sequence with at least one nucleotide in the variant sequence being different from at least one nucleotide of the target sequence. For example, if a wild type mRNA has a certain target sequence for a miRNA, then another mRNA with the wild type sequence but with at least one additional mismatch with the miRNA in the target sequence would be considered to have a variant target sequence.

The term "post transcriptional regulation", as used herein, refers to regulation of protein expression after the gene for the protein has been transcribed into an mRNA.

"Oxidative stress tolerant", with regard to a plant, refers to a plant that is relatively tolerant to oxidative stresses. For example, a transgenic plant expressing more CSD activity than the wild type plant from which it was derived is shown herein to be more tolerant of oxidative stress challenges than the wild type.

### DESCRIPTION OF THE DRAWINGS

Figure 1 shows schematic diagrams and assay results for miR398 targeting of CSD1 and CSD2 mRNA for degradation. 1A shows a phylogenetic tree of the Arabidopsis superoxide dismutase gene family, showing miR398-regulated Cu/Zn-SODs. 1B and 1C show miR398 complementarity to CSD1 and CSD2 mRNA that results in cleavage as determined by RNA ligase-mediated 5' RACE. The frequency of 5'RACE clones corresponding to the cleavage sites are indicated. 1D and 1E show coexpression of miR398 and wild-type CSD1 and CSD2 constructs in *Nicotiana bentahmiana.* Northern blot analysis was performed with the RNA extracted from leaves collected 2 days after infection with Agrobacteria and probed with CSD1 or CSD2.

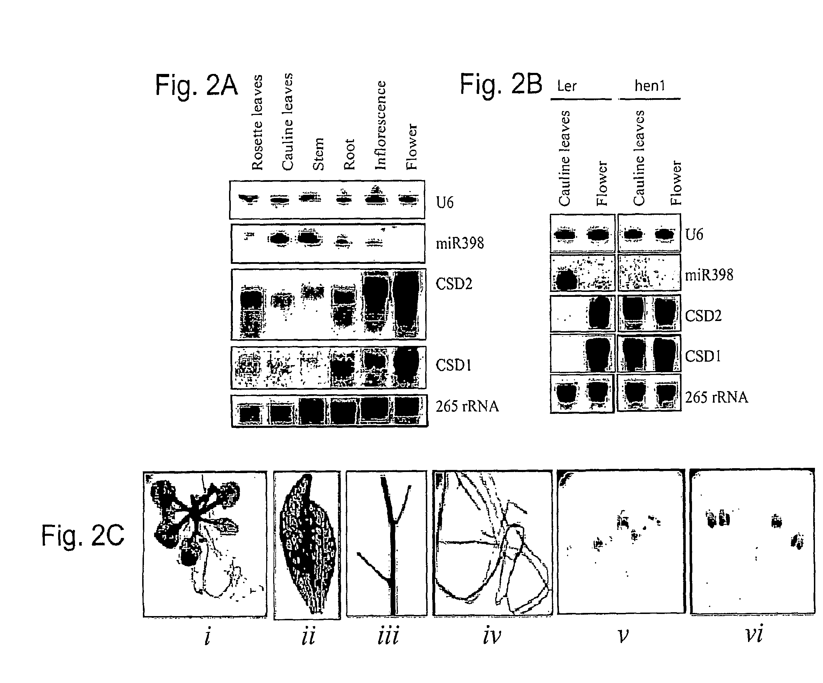

Figure 2 shows miR398, CSD1 and CSD2 expression patterns in plants. 2A shows tissue expression patterns. For small RNA blots, 10 *µ*g of total RNA was loaded and the blot probed for miR398 or U6 RNA (as a loading control). For high-molecular-weight RNA, 20 *µ*g of total RNA was loaded and the blot probed with full-length cDNA probes of the CSD1 and CSD2. The blot re-probed with 26S rRNA is shown as a loading control. 2B shows miR398, CSD1 and CSD2 expression in indicated tissues of henl-1 mutant and the wild-type Landsberg erecta (Ler). For small RNA blots, 10 *µ*g of total RNA was loaded and the blot probed for miR398 or U6 RNA (as a loading control). For high-molecular-weight RNA, 20 *µ*g of total RNA was loaded and the blot probed with full-length cDNAs of the CSD1 and CSD2. The blot re-probed with 26S rRNA is shown as a loading control. 2C shows three-week-old representative transgenic Arabidopsis line carrying a 2.0-kb miR398b promoter-GUS construct processed for histochemical GUS staining. ß-Glucoronidase activity is visualized by the blue color. The staining is mainly observed in the vascular tissue of leaves (i and ii), root of GUS-stained Arabidopsis seedling (iii). The staining is visible in the primary root as well as in the secondary roots. The staining is also seen in stem (iv). Inflorescence with anthers stained (v) and a closer view of a flower to visualize anther staining (vi).

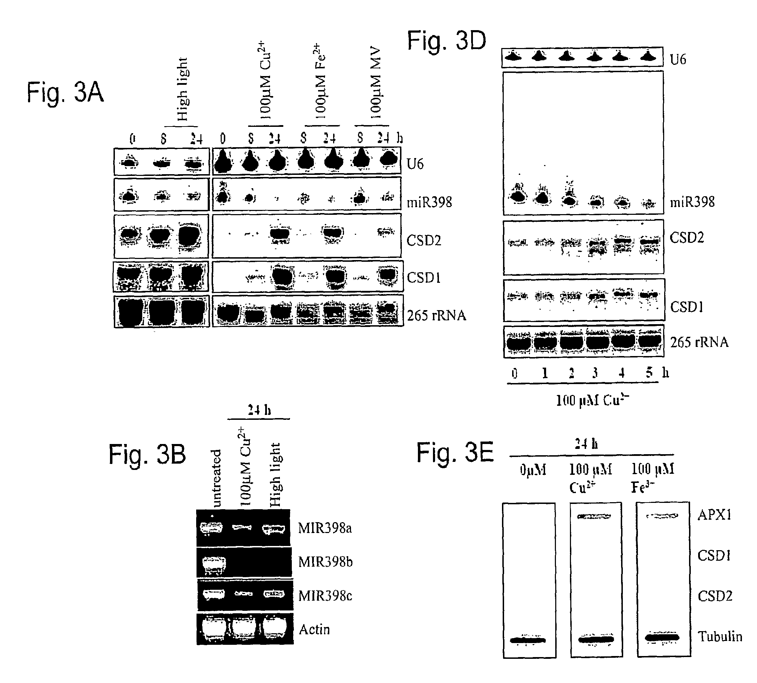

Figure 3 shows miR398, CSD1 and CSD2 expression in response to high light, Cu²⁺, Fe³⁺ and methyl viologen treatments. 3A shows miR398, CSD1 and CSD2 in response to high light, Cu²⁺, Fe³⁺ and methyl viologen treatment. Each lane contained 10 *µ*g (miR398 analysis or CSD1 and CSD2 analysis) of total RNA isolated from 15 day-old wild-type seedlings either transferred to high light (800 *µ*mol m⁻² S⁻¹) or sprayed with 100 *µ*M Cu²⁺ or 100 *µ*M Fe³⁺ and seedlings were harvested after 8 and 24 h of treatment. RNA blot analysis was performed as indicated for Figure 2. 3B shows RT-PCR analyses of precursor transcripts of miR398 family members in response to stress. Total RNA isolated from 15 day-old wild-type seedlings either transferred to high light (800 *µ*mol m⁻² s⁻¹) or sprayed with 100 *µ*M Cu²⁺ and seedlings were harvested after 24 h of treatment. Actin served as a loading control. 3C shows a time-course of miR398, CSD1 and CSD2 expression pattern in response to 100 *µ*M Cu²⁺ treatment. RNA blot analysis was performed as indicated in Figure 2. 3D shows three-week-old wild-type seedlings assayed by nuclear run-on to determine the CSD1 and CSD2 transcriptional response to 100 *µ*M Cu²⁺ or 100 *µ*M Fe³⁺ treatment after 24 h.

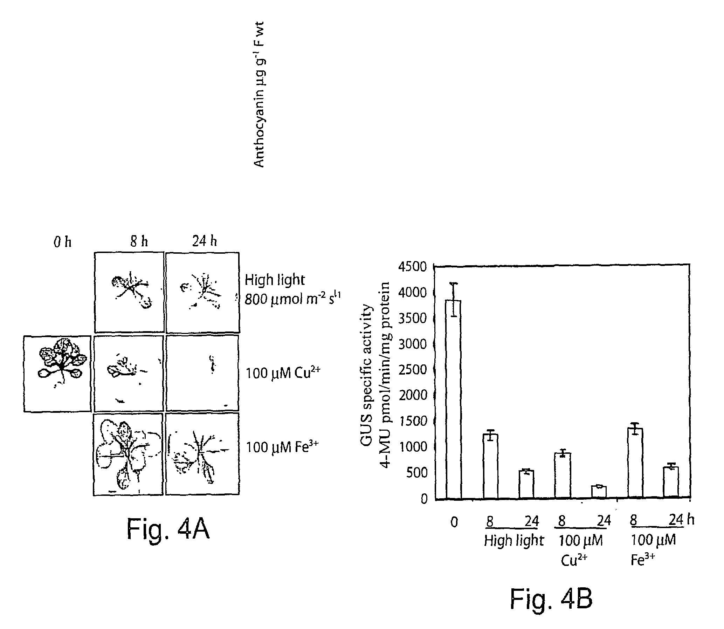

Figure 4 shows the response of miR398b promoter::GUS during high light, Cu²⁺ or Fe³⁺ treatments. 4A shows miR398b promoter::GUS staining. Three-week-old transgenic seedlings on MS-agar medium were either transferred to high light (800 *µ*mol m⁻²s⁻¹) or sprayed with 100 *µ*M Cu²⁺ or 100 *µ*M Fe³⁺. After 24 h of exposure the seedlings were stained for GUS activity. 4B shows quantification of ß-glucoronidase activity in 3-week-old transgenic seedlings grown on MS-agar medium either transferred to high light (800 *µ*mol m⁻² S⁻¹) or sprayed with 100 *µ*M Cu²⁺ or 100 *µ*M Fe³⁺, and the GUS activity was assayed after 24 h of treatment. The results are mean of GUS activities from 3 independent experiments. Specific GUS activities are expressed as pmol of 4-methylunbelliferone per mg of total protein per min.

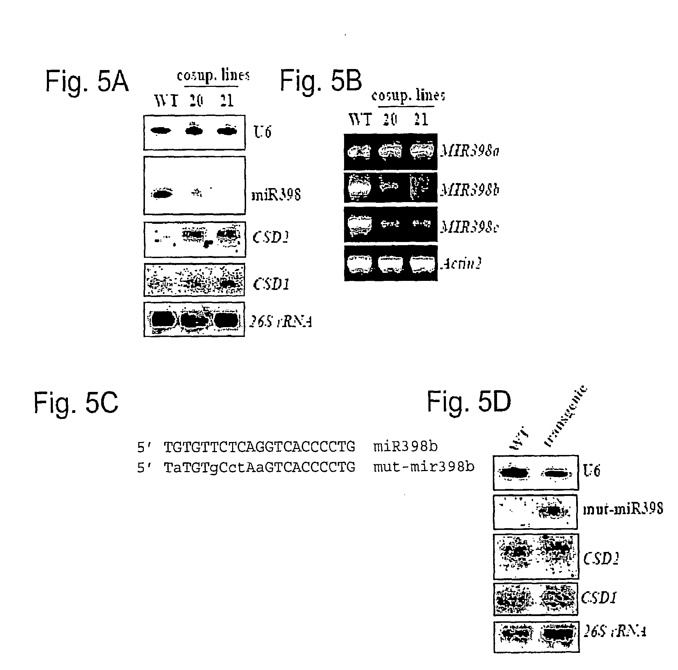

Figure 5 shows co-suppression of CSD1 and CSD2 by miR398. 5A shows northern blot analysis of representative transgenic lines of a miR398 overexpression constructs. RNA blot analysis was performed as indicated in Figure 2. 5B shows RT-PCR analyses of precursor transcripts of MIR398 family members in co-suppression lines. Actin served as a loading control. 5C shows introduced mutations in the mut-miR398b in lower case letters. 5D shows overexpression of mutated miR398b (mut-miR398b) in transgenic plants. Northern analysis of representative transgenic lines. RNA blot analysis was performed as indicated for Figure 2.

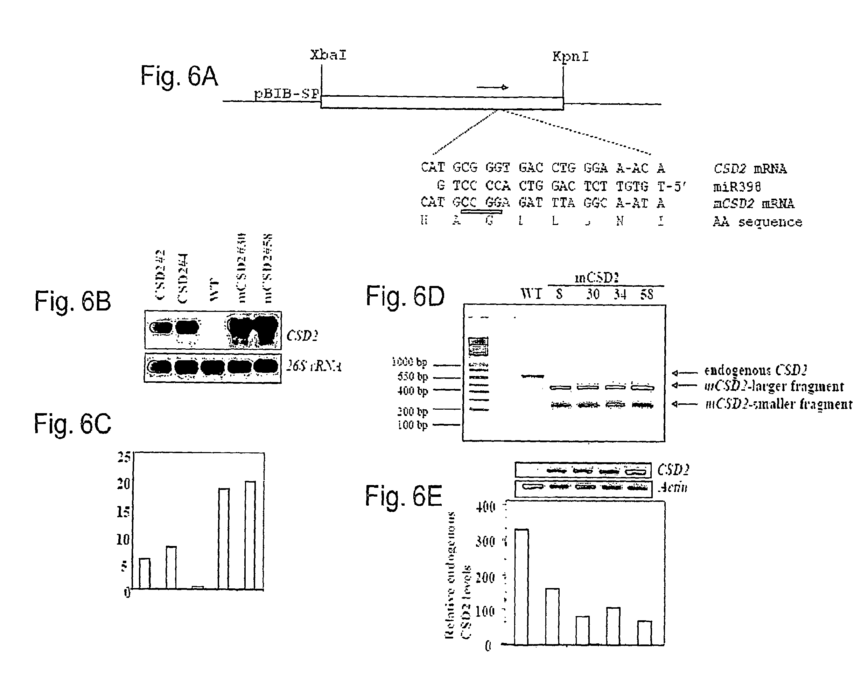

Figure 6 shows CSD2 expression analysis in CSD2 and mCSD2 transgenic lines and wild-type plants. 6A shows a CSD2 construct in pBIB binary vector. The expanded sequence shows CSD2 and mCSD2 mRNA sequences correspondence to the miR398 sequence. A point mutation introduced in the miR398 complementary sequence created an Msp1 site and is underlined. 6B show RNA blot analysis of CSD2 expression with 10 *µ*g of total RNA isolated from CSD2 or mCSD2 transgenic and wild-type plants. 6C shows expression levels quantified by use of a phosphoimager and Imagequant software. 6D shows a determination of endogenous CSD2 levels in mCSD2 transgenic lines and in the wild-type plants. Endogenous and miRNA-resistant (mCSD2) transcripts were amplified by RT-PCR and distinguished by digestion with the restriction enzyme Msp1, which cuts only the mutant form. Endogenous CSD2 transcript is decreased substantially in mCSD2 plants, which indicates a feedback regulation of CSD2. Agarose-gel separation and ethidium-bromide staining revealed the full-length PCR product (651 bp) and the Msp1 digestion fragments (428 bp and 223 bp). The bottom histogram panel shows the CSD2 expression levels in mCSD2 transgenic lines as determined by RT-PCR. As a control, Actin2 fragment was amplified. 6E shows quantification of endogenous CSD2 levels in mCSD2 transgenic plants and wild-type plants with use of a phosphoimager and Imagequant software.

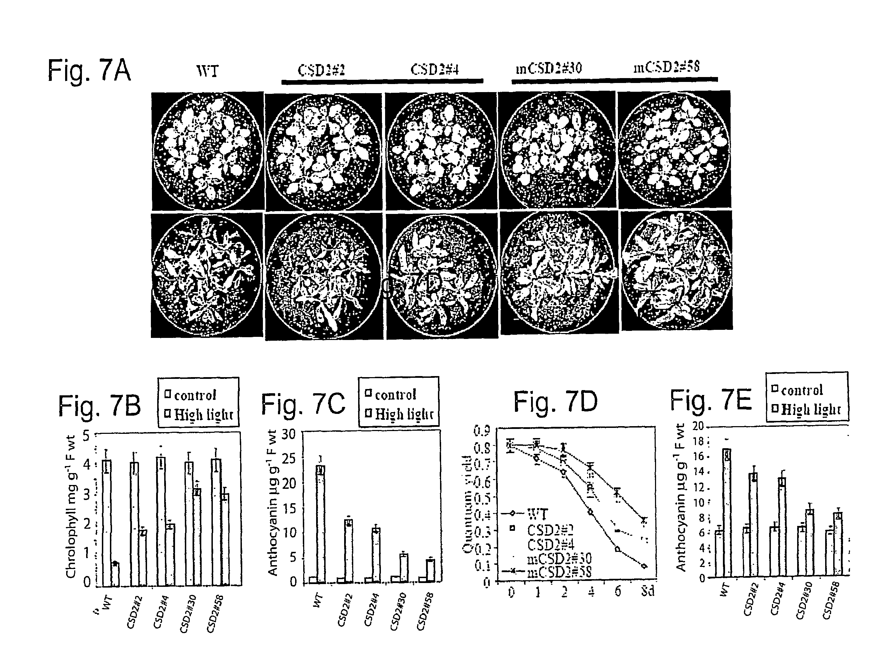

Figure 7 shows a response of transgenic and wild-type plants to high light stress treatment. Top panel 7A shows plants grown under normal light intensity (100 µmol m⁻² s⁻¹) throughout the experimentation. 7A bottom panel shows CSD2 and mCSD2 transgenic lines and the wild-type exposed to continuous high light intensity (800 *µ*mol m⁻² s⁻¹). The photographs were taken after 8 days of exposure. 7B shows chlorophyll and 7C shows anthocyanin content in leaves with or without high light for 8 days. Data are the mean ± SD of 3 independent experiments. 7D shows changes of quantum yield in CSD2 and mCSD2 transgenic lines and the wild type during high light stress. Data are the mean ± SD of 3 independent experiments. 7E shows lipid peroxidation expressed as MDA content in seedlings of CSD2 and mCSD2 transgenic lines and the wild type after 8 days of exposure to high light stress. Data are the mean ± SD of 3 independent experiments.

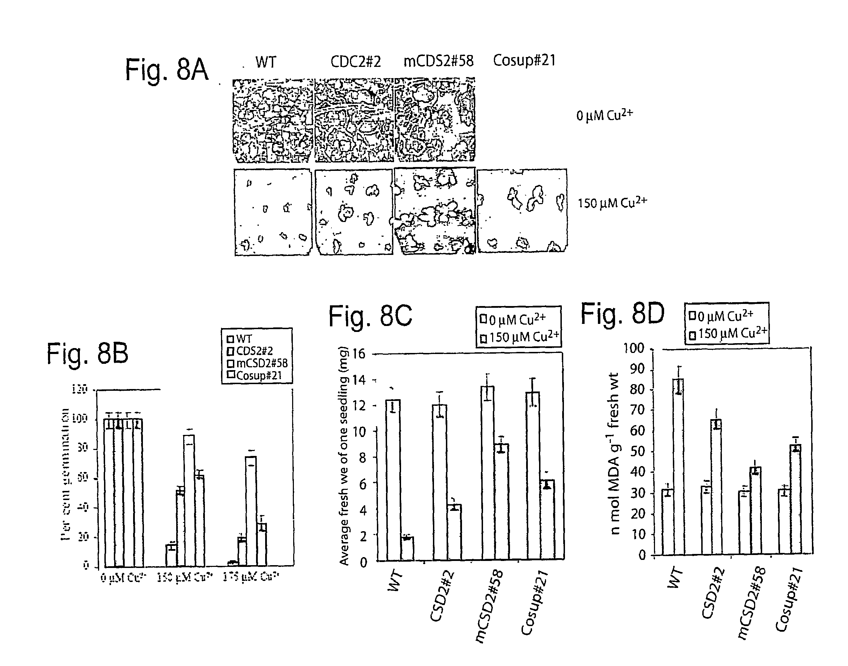

Figure 8 shows a response of transgenic and wild-type plants to Cu²⁺. 8A shows germination and seedling development of CSD2, mCSD2 transgenic and miR398 cosuppression lines and the wild type exposed to 0 or 150 *µ*M Cu²⁺ stress. Photographs were taken after 18 days' exposure to Cu²⁺. 8B shows germination scored when the radicle tips had fully emerged from the seed coats. Data are the means ± standard deviations of 3 independent experiments (30 seeds/genotype/experiment). 8C shows the average fresh weight of seedlings grown on MS-agar plates containing 0 or 150 *µ*M Cu²⁺ for 18 days. For each data point, 30 seedlings were collected and weighed. The results are presented as average fresh weight per seedling. Data are the mean ± SD of 3 independent experiments. 8D shows lipid peroxidation expressed as MDA content in seedlings of CSD2, mCSD2 transgenic, miR398 cosuppression lines and the wild type after 18 days' exposure to 150 *µ*M Cu²⁺, Data are the mean ± SD of 3 independent experiments.

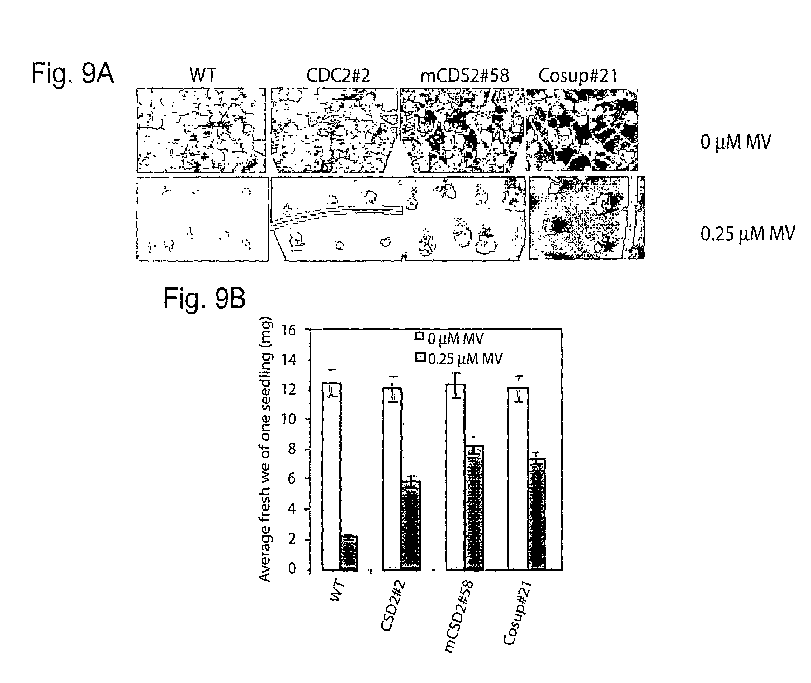

Figure 9 shows a response of transgenic and the wild-type plants to MV treatment. 9A shows germination and seedling development of CSD2 and mCSD2 transgenic and miR398 cosuppression lines and the wild type exposed to 0 or 0.25 *µ*M MV containing MS-agar plates. Photographs were taken after 18 days' exposure to MV. 9B shows the fresh weight of 30 seedlings grown under indicated concentrations of MV for 18 days. The results are presented as the average fresh weight of 30 seedlings for each data point. Data are the mean ± SD of 3 independent experiments.

### DETAILED DESCRIPTION

Cellular superoxide dismutase activity increases in response to oxidative stress in plants. Superoxide anion radicals (O₂⁻) can be formed as a result of general cell metabolism and can be induced by outside factors, such as light or chemicals. The highly reactive superoxide radicals can cause any number of harmful reactions with cell molecules. Some protection from the superoxide radicals can be provided by superoxide dismutase enzymes that catalyze the reaction:

Cu²⁺-SOD + O₂⁻ → Cu¹⁺-SOD + O₂

followed by:

Cu¹⁺-SOD + O₂ + 2H⁺ → Cu²⁺-SOD + H₂O₂.

In particular, copper/zinc superoxide dismutase (CSD) activity appears to increase in response to oxidative stresses. The increased activity is due to accumulation of constitutively expressed mRNA for the enzyme. We have found that this increase is due to down regulation of a micro-RNA that targets a region of the CSD for cleavage.

Furthermore, we have determined a variety of CSD mutations that can liberate the superoxide dismutase system from regulation by the miR398. The regulatory nucleic acid miR398 is highly conserved across all plant species, e.g., from rice, to cotton, soybean, *Medicago* species, and the like. We have found that introduction of CSD1 and/or CSD2 sequences containing miR398 target sequences having additional mismatches can enhance a plant's resistance to oxidative stress. For example, introduction of CSD1 genes with variations on the sequence A AGG GGT TTC CTG AGA TCA CA (SEQ ID NO: 1), or introduction of CSD2 genes with variations on the sequence T GCG GGT GAC CTG GGA AAC A (SEQ ID NO: 2), can be markedly resistant to the stress of high intensity light, heavy metals, and oxidative chemicals.

### Micro-RNA

Short RNAs called microRNAs (miRNAs) have been identified in a variety of species. Typically, these endogenous RNAs are each transcribed as a long RNA and then processed to a pre-miRNA of approximately 60-75 nucleotides that forms an imperfect hairpin (stem-loop) structure. The pre-miRNA is typically then cleaved, e.g., by Dicer, to form the mature miRNA. Mature miRNAs are typically approximately 21-25 nucleotides in length, but can vary, e.g., from about 14 to about 25 or more nucleotides. Some, though not all, miRNAs have been shown to inhibit translation of mRNAs bearing partially complementary sequences. Such miRNAs contain one or more internal mismatches to the corresponding mRNA that are predicted to result in a bulge in the center of the duplex formed by the binding of the miRNA antisense strand to the mRNA. The miRNA typically forms approximately 14-17 Watson-Crick base pairs with the mRNA; additional wobble base pairs can also be formed. In addition, short synthetic double-stranded RNAs (e.g., similar to siRNAs) containing central mismatches to the corresponding mRNA have been shown to repress translation (but not initiate degradation) of the mRNA. See, for example, Zeng et al. (2003) "MicroRNAs and small interfering RNAs can inhibit mRNA expression by similar mechanisms" Proc. Natl. Acad. Sci. USA 100:9779-9784; Doench et al. (2003) "siRNAs can function as miRNAs" Genes & Dev. 17:438-442; Bartel and Bartel (2003) "MicroRNAs: At the root of plant development?" Plant Physiology 132:709-717; Schwarz and Zamore (2002) "Why do miRNAs live in the miRNP?" Genes & Dev. 16:1025-1031; Tang et al. (2003) "A biochemical framework for RNA silencing in plants" Genes & Dev. 17:49-63; Meister et al. (2004) "Sequence-specific inhibition of microRNA- and siRNA-induced RNA silencing" RNA 10:544-550; Nelson et al. (2003) "The microRNA world: Small is mighty" Trends Biochem. Sci. 28:534-540; Scacheri et al. (2004) "Short interfering RNAs can induce unexpected and divergent changes in the levels of untargeted proteins in mammalian cells" Proc. Natl. Acad. Sci. USA 101:1892-1897; Sempere et al. (2004) "Expression profiling of mammalian microRNAs uncovers a subset of brain-expressed microRNAs with possible roles in murine and human neuronal differentiation" Genome Biology 5:R13; Dykxhoorn et al. (2003) "Killing the messenger: Short RNAs that silence gene expression" Nature Reviews Molec. and Cell Biol. 4:457-467; McManus (2003) "MicroRNAs and cancer" Semin Cancer Biol. 13:253-288; and Stark et al. (2003) "Identification of Drosophila microRNA targets" PLoS Biol. 1:E60.

The cellular machinery involved in translational repression of mRNAs by partially complementary RNAs (e.g., certain miRNAs) appears to partially overlap that involved in RNAi, although, as noted, translation of the mRNAs, not their stability, is affected and the mRNAs are typically not degraded.

The location and/or size of the bulge(s) formed when the antisense strand of the RNA binds the mRNA can affect the ability of the RNA to repress translation of the mRNA. Similarly, location and/or size of any bulges within the RNA itself can also affect efficiency of translational repression. See, e.g., the references above. Typically, translational repression is most effective when the antisense strand of the RNA is complementary to the 3' untranslated region (3' UTR) of the mRNA. Multiple repeats, e.g., tandem repeats, of the sequence complementary to the anti sense strand of the RNA can also provide more effective translational repression; for example, some mRNAs that are translationally repressed by endogenous miRNAs contain 7-8 repeats of the miRNA binding sequence at their 3' UTRs. It is worth noting that translational repression appears to be more dependent on concentration of the RNA than RNA interference does; translational repression is thought to involve binding of a single mRNA by each repressing RNA, while RNAi is thought to involve cleavage of multiple copies of the mRNA by a single siRNA-RISC complex.

Guidance for design of a suitable RNA to repress translation of a given target mRNA can be found in the literature (e.g., the references above and Doench and Sharp (2004) "Specificity of microRNA target selection in translational repression" Genes & Dev. 18:504-511; Rehmsmeier et al. (2004) "Fast and effective prediction of microRNA/target duplexes" RNA 10:1507-1517; Robins et al. (2005) "Incorporating structure to predict microRNA targets" Proc Natl Acad Sci 102:4006-4009; and Mattick and Makunin (2005) "Small regulatory RNAs in mammals" Hum. Mol. Genet. 14:R121 - R132, among many others) and herein.

miR398 is one of the recently discovered microRNAs in Arabidopsis and rice, and it is also conserved in other flowering plants (Bonnet et al., 2004; Jones-Rhoades and Bartel, 2004; Sunkar and Zhu, 2004; Axtell and Bartel, 2005; Sunkar et al., 2005). miR398 targets 2 closely related Cu/Zn-superoxide dismutases, the cytosolic CSD1 (At1g08830) and plastidic CSD2 (At2g28190) (see Figure 1A; Klebenstein et al., 1998; Bonnet et al., 2004; Jones-Rhoades and Bartel, 2004). Figures 1B and 1C show the sites of miR398-directed cleavage of the CSD1 and CSD2 transcripts, respectively, as detected by a modified 5' RACE assay (Llave et al., 2002). Data herein demonstrate a regulatory relationship between miR398 and its target genes, CSD1 and CSD2, in a transient coexpression assay in *Nicotiana benthamiana* leaves. After 2 days of coexpression with miR398, CSD1 and CSD2 mRNA decreased substantially, which demonstrated that the miR398 can direct the degradation of CSD1 and CSD2 mRNA in vivo (Figures 1D and 1E).

Described herein for the first time is a miRNA that is down-regulated by stress. In particular, we have found that the expression of miR398 is down-regulated by oxidative stress. This down-regulation is important for the posttranscriptional induction of CSD1 and CSD2 expression under oxidative stress conditions. Furthermore, we show that relieving miRNA-directed silencing, e.g., by overexpression of a miR398-resistant version of CSD2 gene, leads to great improvement of plant resistance to oxidative stress conditions such as high light, heavy metal and methyl viologen. Thus, our findings provide the first evidence that suppressing the expression of a miRNA is important for plant adaptation to abiotic stresses. miR398 targets two closely related Cu/Zn-superoxide dismutases (cytoslic-CSD1 and chloroplastic-CSD2) that can detoxify superoxide radicals. CSD1 and CSD2 transcripts are induced in response to oxidative stress, but the regulatory mechanism of the induction is unknown.

miR398 expression is downregulated transcriptionally by oxidative stresses, and this down-regulation is important for posttranscriptional CSD1 and CSD2 mRNA accumulation, translation and expression of oxidative stress tolerance. An important role of miR398 in specifying the spatial and temporal expression patterns of CSD1 and CSD2 mRNA is identified herein. For example, Experimental results indicate that CSD1 and CSD2 expression is fine-tuned by miR398-directed mRNA cleavage. Transgenic Arabidopsis plants overexpressing a miR398-resistant form of CSD2 are shown herein to accumulate more CSD2 mRNA than plants overexpressing a regular CSD2 and are consequently much more tolerant to high light, heavy metals and other oxidative stresses. Thus, we show that relieving miR398-guided silencing of CSD2 in transgenic plants is an effective new approach to improving plant productivity under oxidative stress conditions.

Plant miRNAs generally direct their target mRNAs for endonucleolytic cleavage (Llave et al., 2002; Tang et al., 2003; Mallory et al., 2004; 2005; Allen et al., 2005; Axtell and Bartel, 2005; Guo et al., 2005; Schwab et al., 2005; Sunkar et al., 2005). A negative correlation between the expression of a miRNA and its target mRNAs is expected within a given tissue or organ. The expression profile of miR398, CSD1 and CSD2 in the same RNA samples indicates a clear negative correlation and suggests a critical role for miR398 in controlling the CSD1 and CSD2 mRNA levels in different tissues, organs or developmental stages in Arabidopsis. The observation that miR398 determines the expression pattern of CSD1 and CSD2 is supported by the analysis of miRNA biosynthetic mutant hen1-1 in which miR398 expression is impaired (Figure 2B).

Although the precise physiological implication for the differential accumulation of CSD1 and CSD2 mRNA in different tissues or organs was not previously known, some tissues likely require a high level of CSD1 and CSD2 expression even under normal growth conditions. This notion is consistent with our finding that constitutively overexpressing miR398 is impossible, probably because that such overexpression may lead to a general silencing of CSD1 and CSD2 in all tissues, which might be lethal to plants. Rhizsky et al (2003) have also suggested that a complete knockout of CSD2 may be lethal.

It is well established that cells regulate the expression of many stress-inducible genes at the level of transcription (Kawasaki et al., 2001; Seki et al. 2001; Fowler and Thomashow, 2002; Zhu, 2002). Also, some of the stress-inducible genes might be regulated at the post-transcriptional levels, although the underlying mechanisms are poorly understood (Derocher and Bohnert 1993; Cohen et al. 1999; Kawaguchi et al. 2004). In the present disclosure, it is shown for the first time that stress induction of genes can be mediated by the down-regulation of a miRNA.

Environmental stress conditions such as drought, salinity, high light or heavy metals cause a rapid and excessive accumulation of reactive oxygen species (ROS) in plant cells (Hasegawa et al., 2000; Zhu, 2002; Apel and Hirt, 2004; Bartels and Sunkar, 2005). Superoxide dismutases (SODs; EC 1.15.1.1) represent the first line of defense against superoxide accumulation by rapidly converting superoxide to hydrogen peroxide (H₂O₂) and molecular oxygen (Fridovich, 1995). Cu/Zn SODs are arguably the most important SODs, and their roles in plant stress responses are supported by their increased expression under stress and by the phenotypic analysis of a CSD2 knock-down mutant. CSD1 and CSD2 mRNA is induced under oxidative stress conditions (Figure 3A; Perl-Treves and Galun, 1991; Tsang et al., 1991; Kurepa et al., 1997; Klebenstein et al., 1998). The present results suggest that CSD1 and CSD2 induction by oxidative stress conditions depends on the suppression of miR398. CSD1 and CSD2 transcription did not differ between control and Cu²⁺ or Fe³⁺ treatments as indicated by nuclear-run on assays (Figure 3B). Therefore, CSD1 and CSD2 regulation under oxidative stress occurs at the posttranscriptional level and occurs by suppression of miRNA expression, thus relieving its silencing effect on CSD1 and CSD2 mRNAs. This is the first demonstration that a plant microRNA is a direct target of oxidative stress signaling, and its expression level and miR398b promoter activity are suppressed under stress conditions.

Furthermore, our ability to distinguish the endogenous CSD2 mRNA from that of our miR398-resistant version of CSD2 (mCSD2 mRNA), allowed us to decipher the existence of a feedback regulatory control (Figure 6D). The observation that endogenous CSD2 mRNA is subjected to negative feedback regulation suggests the need for homeostasis of this gene product. Feedback regulation may provide a sensitive mechanism for fine-tuning CSD2 gene expression.

Several attempts have been made to improve plant stress tolerance by overproduction of Cu/Zn-SODs in transgenic plants (Sen Gupta et al., 1993a and 1993b; Perl et al., 1993; Tepperman and Dunsmuir, 1990). The introduced genes contained the miR398 target sites in their ORFs, and as shown here, were most likely negatively influenced by the miR398 present in wild-type plants, which can explain why minimal or no increases in stress tolerance were observed in some of the studies (Tepperman and Dunsmuir, 1990; Pitcher et al., 1991). As shown here in experiments with high light, Cu²⁺, Fe³⁺ and MV stress, introducing a CSD2 gene with the miR398 recognition site destroyed can produce a substantial increase in tolerance. These results suggest that understanding posttranscriptional gene regulation is important for our ability to manipulate stress tolerance in plants. Because miR398 is conserved in crop plants (Bonnet et al., 2004; Jones-Rhoades and Bartel, 2004; Sunkar and Zhu, 2004; Sunkar et al., 2005; Lu et al., 2005), our findings offer an improved strategy to engineer crop plants with enhanced stress tolerance.

We note that the solution to the problem of effectively enhancing CSD activity is not to increase the amount of CSD encoding DNA, but to provide a CSD with reduced sensitivity to miR398 regulation. There is a 4 nt complementarity mismatch between miR398 and the target sequence of CSD2, and a 5 nt complementarity mismatch between miR398 and the target sequence of CSD1. miR398 binds to a complimentary target region in the CSDs of only 21 nt and we have found that further mismatches can substantially reduce the ability of miR398 to regulate CSD activity.

### Introduction of mismatches into miRNA targets

A variety of strategies can be employed to effectively protect plants under stress using the compositions and methods of the present invention. Typically, a superoxide dismutase gene having a variant miR398 target sequence is designed and constructed; the gene is placed into an appropriate expression vector construct; plant cells are transfected or transformed with the expression construct; and, the cells are cultured in vitro or propagated as living plants.

Genes having wild type miR398 target sequences are obtained in any fashion known in the art. For example, the original sequences can be reverse transcribed to the form of a cDNA, e.g., obtained by RNA ligase mediated rapid amplification of cDNA ends (LM-RACE). Optionally, the wild type sequences can be obtained from a gene bank library, cloned directly from a plant genome, or synthesized using a DNA synthesizer based on a known sequence. With regard to the present invention, the preferred genes are copper/zinc superoxide dismutase (CSD) or cytochrome C oxidase genes (e.g., Cytochrome C oxidase subunit V (At3g15640) in plants. The most preferred genes, post transcriptionally regulatable by miR398, include plant CSD1 and CSD2 genes. The miR398 target sequences in the CSD1 and CSD2 genes are highly conserved across all plants, monocot and dicot, so that the compositions and methods of the present invention should be applicable for all plants, such as grasses, grains, fruiting plants, timber, vegetables, and the like.

The miR398 target sequence of the of a gene can be varied according to techniques known in the art to provide variant sequences less sensitive to regulation by protein complexes associated with miR398. A gene including a variant target sequence can be synthesized directly on an automated DNA synthesizer. Alternately, point mutations can be introduced into a cloned wild type CSD target sequence, e.g., by site directed mutagenesis or PCR in the presence of primers containing one or more mismatched bases. Optionally, the variant target sequence can be introduced by restriction of the wild type target sequence out of a regulated protein sequence followed by insertion and ligation of the variant target sequence into the protein sequence by classic recombinant genetic engineering techniques.

In preferred embodiments of the invention, the original wild type gene is a plant CSD1 or CSD2 sequence comprising a miR398 target sequence of A AGG GGT TTC CTG AGA TCA CA (SEQ ID NO: 1), or T GCG GGT GAC CTG GGA AAC A (SEQ ID NO: 2), respectively. The reading frame for translation for such sequences it indicated by the spacing between groups with the groups of three nucleotides representing a codon encoding an amino acid.

Useful variants of the miR398 CSD target sequence can include any that significantly reduce the post transcription down regulation of CSD expression associated with miR398-protein complexes. For example, the variants can include SEQ ID NO: 1 or 2 with one or more nucleotides replaced with a different nucleotide. The different nucleotide can be a natural nucleotide (e.g., A, T, G or G) or an unnatural nucleotide, such as a synthetic nucleotide analog capable of acting as a substrate in a nucleotide polymerase reaction. The variants can include replacement of 1, 2, 3, 4, 5, 6, 7, 8, 9, 10, 11, 12, 13, 14, 15, 16, 17, 18, 19, 20, or more nucleotides in the target sequence. The variants can include replacement nucleotides that do not change the amino acid encoded by the triplet codon, e.g., with any of TCT, TCC, TCA, TCG, AGT, or AGC encoding the amino acid serine; GGT, GGC, GGA or GGG encoding glycine, CGT, CGC. CGA, CGG, AGA, or AGG encoding arginine; CAC or CAT encoding histidine; GCT, GCA, GCC, or GCG encoding alanine; GGA, GGC, GGG, or GGT encoding glycine; GAC or GAT encoding aspartate; TTA, TTG, CTT, CTA, CTC, or CTG encoding leucine; AAC or AAT encoding asparagine; TTC or TTT encoding phenylalanine; and/or the like. In some embodiments, the CSD target sequence can include variant codons that change the encoded amino acid, preferably without affecting the activity of the enzyme. In some embodiments, the CSD target sequence includes one or more variant codons that encode an amino acid which is a conservative variant of the corresponding wild type amino acid. In some embodiments, the variants can be at any point along the target sequence without affecting enzymatic activity. For example, where the target is in an untranslated upstream region (UTR), condon degeneracy and/or retention of conservative variant amino acids is not an important consideration in choice of variant mutations. miR398 target sequences on CSD1 mRNA is located in the 5'UTR and therefore translation of codons to amino acid sequences should not be a consideration in selection of miR398 resistant variants.

Owing to the degeneracy of the genetic code, "silent substitutions" (i.e., substitutions in a nucleic acid sequence which do not result in an alteration in an encoded polypeptide) are an implied feature of every nucleic acid sequence which encodes an amino acid. Similarly, "conservative amino acid substitutions", in one or a few amino acids in an amino acid sequence are substituted with different amino acids with highly similar properties, are also readily identified as being highly similar to a disclosed construct. Such conservative variations of each disclosed sequence are a feature of the present invention.

"Conservative variations" of a particular nucleic acid sequence refers to those nucleic acids which encode identical or essentially identical amino acid sequences (*see,* Table 1 below) or, where the nucleic acid does not encode the exact same an amino acid sequence, to essentially identical sequences. One of skill will recognize that individual substitutions, deletions or additions which alter, add or delete a single amino acid or a small percentage of amino acids (typically less than 20%, 10%, more typically less than 5%, 4%, 2% or 1%) in an encoded sequence are "conservatively modified variations" where the alterations result in the deletion of an amino acid, addition of an amino acid, or substitution of an amino acid with a chemically similar amino acid. Moreover, amino acid variations in regions of an enzyme not necessary to the function of the enzyme can be considered conservative variations. Thus, "conservative variations" of a listed polypeptide sequence of the present invention include substitutions of a small percentage, typically less than 5%, more typically less than 2% or 1%, of the amino acids of the polypeptide sequence, with a conservatively selected amino acid of the same conservative substitution group. Finally, the addition of sequences which do not alter the encoded activity of a nucleic acid molecule, such as the addition of a non-functional sequence or sequences with accessory functions, is a conservative variation of the basic nucleic acid.

**Table 1 -- Conservative Substitution Groups**

| | | | | |
|---|---|---|---|---|
| 1 | Alanine (A) | Serine (S) | Threonine (T) | |
| 2 | Aspartic acid (D) | Glutamic acid (E) | | |
| 3 | Asparagine (N) | Glutamine (Q) | | |
| 4 | Arginine (R) | Lysine (K) | | |
| 5 | Isoleucine (I) | Leucine (L) | Methionine (M) | Valine (V) |
| 6 | Phenylalanine (F) | Tyrosine (Y) | Trytophan (W) | |

In Table 1, substitution of an amino acid with another amino acid of the same group number can be considered a conservative substitution.

In a preferred target sequence for a CSD1 variant (i.e., CSD1 modified to have a variant target sequence), the variant miR398 target sequence can be A AGN GGN TTN CTN AGN TCN CA (SEQ ID NO: 26), wherein N is any nucleotide, and at least one of the Ns represents an nucleotide different from the corresponding nucleotide of SEQ ID NO: 1. In this embodiment, it is preferred that 2, 3, 4, or 5 of the "N" nucleotides are different from the corresponding nucleotides of SEQ ID NO: 1. In a most preferred embodiment, all 6 "N" nucleotides are different nucleotides.

In preferred target sequences for a CSD2 variants, the variant miR398 target sequence is N GCN GGN GAN TTN GGN AAN A (SEQ ID NO: 27) or CAN GCN GGN GAN NTN GGN AAN A (SEQ ID NO: 30), wherein N is any nucleotide, and at least one of the Ns represents an nucleotide different from the corresponding nucleotide of SEQ ID NO: 2. In this embodiment, it is preferred that 2, 3, 4, 5 or 6 of the "N" nucleotides are different from the corresponding nucleotides of SEQ ID NO: 2. In a most preferred embodiment, all "N" nucleotides are different. The sequence CAT GCC GGA GAT TTA GGC AAT A (SEQ ID NO: 5) is a particularly preferred target variant sequence for inclusion in a variant CSD2 sequence.

### Introduction of variant sequences into plants

Once a desired variant CSD sequence has been identified and obtained, it is often useful to incorporate the sequence into a recombinant vector for replication and/or expression. There are several well known methods of introducing nucleic acids into bacterial cells, e.g., for replication, any of which may be used in the present invention. These include: fusion of the recipient cells with bacterial protoplasts containing the DNA, electroporation, projectile bombardment, and infection with viral vectors, etc. Bacterial cells are often used to amplify the number of plasmids containing DNA constructs of this invention. The bacteria are grown to log phase and the plasmids within the bacteria can be isolated by a variety of methods known in the art (see, for instance, Sambrook). In addition, a plethora of kits are commercially available for the purification of plasmids from bacteria. For their proper use, follow the manufacturer's instructions (see, for example, EasyPrep™, FlexiPrep™, both from Pharmacia Biotech; StrataClean™, from Stratagene; and, QIAexpress Expression System™ from Qiagen). The isolated and purified plasmids can then be further manipulated to produce other plasmids, used to transfect plant cells or incorporated into *Agrobacterium tumefaciens* related vectors to infect plants. Typical vectors contain transcription and translation terminators, transcription and translation initiation sequences, and promoters useful for regulation of the expression of the particular nucleic acid. The vectors optionally comprise generic expression cassettes containing at least one independent terminator sequence, sequences permitting replication of the cassette in eukaryotes, or prokaryotes, or both, (e.g., shuttle vectors) and selection markers for both prokaryotic and eukaryotic systems. Vectors are suitable for replication and integration in prokaryotes, eukaryotes, or preferably both. See, Giliman & Smith, Gene 8:81 (1979); Roberts, et al., Nature, 328:731 (1987); Schneider, B., et al., Protein Expr. Purif. 6435:10 (1995); Berger, Sambrook, Ausubel. A catalogue of Bacteria and Bacteriophages useful for cloning is provided, e.g., by the ATCC, e.g., The ATCC Catalogue of Bacteria and Bacteriophage (1992) Ghema et al. (eds) published by the ATCC. Additional basic procedures for sequencing, cloning and other aspects of molecular biology and underlying theoretical considerations are also found in Watson et al. (1992) Recombinant DNA, Second Edition Scientific American Books, NY.

Methods of transducing plant cells with nucleic acids are generally available. In addition to Berger, Ausubel and Sambrook, useful general references for plant cell cloning, culture and regeneration include Payne et al. (1992) Plant Cell and Tissue Culture in Liquid Systems John Wiley & Sons, Inc. New York, NY (Payne); and Gamborg and Phillips (eds) (1995) Plant Cell, Tissue and Organ Culture; Fundamental Methods Springer Lab Manual, Springer-Verlag (Berlin Heidelberg New York) (Gamborg). A variety of Cell culture media are described in Atlas and Parks (eds) The Handbook of Microbiological Media (1993) CRC Press, Boca Raton, FL (Atlas). Additional information for plant cell culture is found in available commercial literature such as the Life Science Research Cell Culture Catalogue (1998) from Sigma-Aldrich, Inc (St Louis, MO) (Sigma-LSRCCC) and, e.g., the Plant Culture Catalogue and supplement (1997) also from Sigma-Aldrich, Inc (St Louis, MO) (Sigma-PCCS).

The nucleic acid constructs of the invention can be introduced into plant cells, either in culture or in the organs of a plant by a variety of conventional techniques. For example, the DNA construct can be introduced directly into the genomic DNA of the plant cell using techniques such as electroporation and microinjection of plant cell protoplasts, or the DNA constructs can be introduced directly to plant cells using ballistic methods, such as DNA particle bombardment. The DNA fragments can be introduced into plant tissues, cultured plant cells or plant protoplasts by standard methods including electroporation (From et al., Proc. Natl. Acad. Sci. USA 82, 5824 (1985), infection by viral vectors such as cauliflower mosaic virus (CaMV) (Hohn et al., Molecular Biology of Plant Tumors, (Academic Press, New York, 1982) pp. 549-560; Howell, US 4,407,956), high velocity ballistic penetration by small particles with the nucleic acid either within the matrix of small beads or particles, or on the surface (Klein et al., Nature 327, 70-73 (1987)), use of pollen as vector (WO 85/01856), or use of *Agrobacterium tumefaciens* or A. *rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al., Science 233, 496-498 (1984); Fraley et al., Proc. Natl. Acad. Sci. USA 80, 4803 (1983)). The virulence functions of the *Agrobacterium tumefaciens* host can direct the insertion of the construct and adjacent marker into the plant cell DNA when the cell is infected by the bacteria.

Microinjection techniques are known in the art and well described in the scientific and patent literature. The introduction of DNA constructs using polyethylene glycol precipitation is described in Paszkowski, et al., EMBO J. 3:2717 (1984). Electroporation techniques are described in Fromm, et al., Proc. Nat'l. Acad. Sci. USA 82:5824 (1985). Ballistic transformation techniques are described in Klein, et al., Nature 327:70-73 (1987).

*Agrobacterium tumefaciens*-mediated transformation techniques, including disarming and use of binary vectors, are also well described in the scientific literature. See, for example, Horsch, et al., Science 233:496-498 (1984), and Fraley, et al., Proc. Nat'l. Acad. Sci. USA 80:4803 (1983). Agrobacterium-mediated transformation is a preferred method of transformation of dicots.

To use isolated sequences corresponding to or linked to expression products in the above techniques, recombinant DNA vectors suitable for transformation of plant cells can be prepared. A DNA sequence coding for the desired mRNA, polypeptide, or non-expressed sequence can be transduced into the plant. Where the sequence is expressed, the sequence is optionally combined with transcriptional and translational initiation regulatory sequences which will direct the transcription of the sequence from the gene in the intended tissues of the transformed plant.

Promoters, in nucleic acids linked to loci identified by detecting expression products, can be identified, e.g., by analyzing the 5' sequences upstream of a coding sequence in linkage disequilibrium with the loci. Sequences characteristic of promoter sequences can be used to identify the promoter. Sequences controlling eukaryotic gene expression have been extensively studied. For instance, promoter sequence elements include the TATA box consensus sequence (TATAAT), which is usually 20 to 30 base pairs upstream of a transcription start site. In most instances the TATA box aids in accurate transcription initiation. In plants, further upstream from the TATA box, at positions -80 to-100, there is typically a promoter element with a series of adenines surrounding the trinucleotide G (or T) N G. See, e.g., J. Messing, et al., in Genetic Engineering in Plants, pp. 221-227 (Kosage, Meredith and Hollaender, eds. (1983)). A number of methods are known to those of skill in the art for identifying and characterizing promoter regions in plant genomic DNA. See, e.g., Jordano, et al., Plant Cell 1:855-866 (1989); Bustos, et al., Plant Cell 1:839-854 (1989); Green, et al., EMBO J. 7:4035-4044 (1988); Meier, et al., Plant Cell 3:309-316 (1991); and Zhang, et al., Plant Physiology 110:1069-1079 (1996).

In construction of recombinant expression cassettes, a plant promoter fragment is optionally employed which directs expression of a nucleic acid in any or all tissues of a regenerated plant. Examples of constitutive promoters include the cauliflower mosaic virus (CaMV) 35S transcription initiation region, the 1'- or 2'- promoter derived from T-DNA of *Agrobacterium tumefaciens,* and other transcription initiation regions from various plant genes known to those of skill. Alternatively, the plant promoter may direct expression of the polynucleotide of the invention in a specific tissue (tissue-specific promoters) or may be otherwise under more precise environmental control (inducible promoters). Examples of tissue-specific promoters under developmental control include promoters that initiate transcription only in certain tissues, such as fruit, seeds, or flowers.

Any of a number of promoters which direct transcription in plant cells can be suitable. The promoter can be either constitutive or inducible. In addition to the promoters noted above, promoters of bacterial origin which operate in plants include the octopine synthase promoter, the nopaline synthase promoter and other promoters derived from native Ti plasmids. See, Herrara-Estrella et al. (1983), Nature, 303:209-213. Viral promoters include the 35S and 19S RNA promoters of cauliflower mosaic virus. See, Odell et al. (1985) Nature, 313:810-812. Other plant promoters include the ribulose-1,3-bisphosphate carboxylase small subunit promoter and the phascolin promoter. The promoter sequence from the E8 gene and other genes may also be used. The isolation and sequence of the E8 promoter is described in detail in Deikman and Fischer, (1988) EMBO J. 7:3315-3327.

If polypeptide expression is desired, a polyadenylation region at the 3'-end of the coding region is typically included. The polyadenylation region can be derived from the natural gene, from a variety of other plant genes, or from T-DNA.

The vector comprising the sequences (e.g., promoters or coding regions) from genes encoding expression products of the invention will typically comprise a nucleic acid subsequence which confers a selectable phenotype on plant cells. The vector comprising the sequence will typically comprise a marker gene, which confers a selectable phenotype on plant cells. For example, the marker can encode biocide tolerance, particularly antibiotic tolerance, such as tolerance to kanamycin, G418, bleomycin, hygromycin, or herbicide tolerance, such as tolerance to chlorosluforon, or phosphinothricin (the active ingredient in the herbicides bialaphos and Basta). For example, crop selectivity to specific herbicides can be conferred by engineering genes into crops which encode appropriate herbicide metabolizing enzymes from other organisms, such as microbes. See, Padgette et al. (1996) "New weed control opportunities: Development of soybeans with a Round UP ReadyTM gene" In: Herbicide-Resistant Crops (Duke, ed.), pp 53-84, CRC Lewis Publishers, Boca Raton ("Padgette, 1996"); and Vasil (1996) "Phosphinothricin-resistant crops" In: Herbicide-Resistant Crops (Duke, ed.), pp 85-91, CRC Lewis Publishers, Boca Raton) (Vasil, 1996). Transgenic plants have been engineered to express a variety of herbicide tolerance/metabolizing genes, from a variety of organisms. For example, acetohydroxy acid synthase, which has been found to make plants which express this enzyme resistant to multiple types of herbicides, has been cloned into a variety of plants (see, e.g., Hattori, J., et al. (1995) Mol. Gen. Genet. 246(4):419). Other genes that confer tolerance to herbicides include: a gene encoding a chimeric protein of rat cytochrome P4507A1 and yeast NADPH-cytochrome P450 oxidoreductase (Shiota, et al. (1994) Plant Physiol. 106(1)17, genes for glutathione reductase and superoxide dismutase (Aono, et al. (1995) Plant Cell Physiol. 36(8):1687, and genes for various phosphotransferases (Datta, et al. (1992) Plant Mol. Biol. 20(4):619. Similarly, crop selectivity can be conferred by altering the gene coding for an herbicide target site so that the altered protein is no longer inhibited by the herbicide (Padgette, 1996). Several such crops have been engineered with specific microbial enzymes for confer selectivity to specific herbicides (Vasil, 1996).

Further, nucleic acids which can be cloned and introduced into plants to modify or complement expression of a gene, including a silenced gene, a dominant gene, and additive gene or the like, can be any of a variety of constructs, depending on the particular application. Thus, a nucleic acid encoding a cDNA expressed from an identified gene can be expressed in a plant under the control of a heterologous promoter. Similarly, a nucleic acid encoding a transcription factor that regulates a target identified by the methods herein, or that encodes any other moiety affecting transcription, can be cloned and transduced into a plant. Methods of identifying such factors are replete throughout the literature. For a basic introduction to genetic regulation, see, Lewin (1995) Genes V Oxford University Press Inc., NY (Lewin), and the references cited therein.

Transformed plant cells which are derived by any of the above transformation techniques can be cultured to regenerate a whole plant which possesses the transformed genotype and thus the desired phenotype. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans, et al., Protoplasts Isolation and Culture, Handbook of Plant Cell Culture, pp. 124-176, Macmillian Publishing Company, New York, (1983); and Binding, Regeneration of Plants, Plant Protoplasts, pp. 21-73, CRC Press, Boca Raton, (1985). Regeneration can also be obtained from plant callus, explants, somatic embryos (Dandekar, et al., J. Tissue Cult. Meth. 12:145 (1989); McGranahan, et al., Plant Cell Rep. 8:512 (1990)), organs, or parts thereof. Such regeneration techniques are described generally in Klee, et al., Ann. Rev. of Plant Phys. 38:467-486 (1987).

These cells can then be cultured into transgenic plants. Plant regeneration from cultured protoplasts is described in Evans et al., "Protoplast Isolation and Culture," Handbook of Plant Cell Cultures 1, 124-176 (MacMillan Publishing Co., New York, 1983); Davey, "Recent Developments in the Culture and Regeneration of Plant Protoplasts," Protoplasts, (1983) pp. 12-29, (Birkhauser, Basal 1983); Dale, "Protoplast Culture and Plant Regeneration of Cereals and Other Recalcitrant Crops," Protoplasts (1983) pp. 31-41, (Birkhauser, Basel 1983); Binding, "Regeneration of Plants," Plant Protoplasts, pp. 21-73, (CRC Press, Boca Raton, 1985).

The CSD2 target for miR398 is conserved, e.g., in rice, maize, barley, wheat, tomato, cotton, sunflower, and more. CSD genes with variant miR398 target sequences of the invention can be usefully introduced into plants, such as, e.g., monocots, dicots, grasses, grains, fruit plants, vegetable plants beans, berries, rice, wheat, corn, oats, barley, alfalfa, soy beans, peanuts, apples, melons, cherries, carrots, tobacco, grapes, lettuce, onions, potatoes, cotton, tomato, and the like. Tobacco and peanuts, for example, can be protected from oxidative stress by incorporation of superoxide dismutase genes or the invention. Each of tobacco *(N. plumbaginifolia)* and peanut (*Arachis hypogaea*) include a CSD1 miR398 target sequence of: AGG GGT TCC CTG AGA TCA CA (SEQ ID NO: 28). Such a sequence can be modified, according to the methods of the invention to reduce the down-regulation of miR398 in these plants.

One of skill will recognize that after the expression cassette is stably incorporated in transgenic plants and confirmed to be operable, it can be introduced into other plants by sexual crossing. Any of a number of standard breeding techniques can be used, depending upon the species to be crossed.

### Oxidative stress suppresses miR398 expression

The CSD1 and CSD2 transcripts were thought to be induced by oxidative stress (Perl-Treves and Galun, 1991; Tsang et al., 1991; Klebenstein et al., 1998), although the mechanism of this induction has been unknown. We investigated whether the level of miR398 that targets CSD1 and CSD2 mRNAs might be altered under oxidative stress conditions. Two-week-old wild-type seedlings grown under regular intensity light (100 *µ*mol m⁻² s⁻¹) were exposed to high light (800 *µ*mol m⁻² s⁻¹) for 8 or 24 h. The miR398 level was down-regulated at 8 h and the signal decreased further with longer treatment (Figure 3A). To further test miR398 regulation by oxidative stress, miR398 expression was studied in seedlings exposed to Cu²⁺, Fe³⁺ and methyl viologen (MV). Heavy metals such as Cu²⁺ and Fe³⁺ are involved in Fenton-type reactions and have a potential to generate hydroxyl radicals (Dietz *et al.*, 1999; Estevez et al., 2001; Babu et al., 2003). MV binds to thylakoid membranes of the chloroplast and transfers the electrons to O₂ in a chain reaction causing continuous formation of superoxide radicals in the presence of light (Asada, 1996). RNA blot analysis showed that miR398 expression was decreased after 8 h of the stress treatment, and the levels were greatly reduced after 24 h of treatment (Figure 3A).

The miR398 family is represented by two members with three loci (MIR398a, MIR398b and MIR398c) in *Arabidopsis* (Bonnet et al., 2004; Jones-Rhoades and Bartel, 2004; Sunkar and Zhu, 2004). miR398b and miR398c are identical in sequence, while miR398a differs from miR398b and miR398c only in its last nucleotide (a thymidine triphosphate in miR398a and a guanine triphosphate in miR398b and miR398c). Using miR398b/miR398c or miR398a probes we detected similar patterns of expression under stress conditions (not shown). These results suggested that the miR398 family members cannot be differentiated in a small RNA blot analyses because of a potential cross-Hybridization problem. To gain insights into which miR398 loci are responsive to oxidative stress conditions, RT-PCR analysis were performed using locus-specific primers designed to amplify precursor transcript including the precursor fold-back sequence in Arabidopsis. In a recent study, Xie et al (2005) provided evidence for the expression of miR398b and miR389c but not miR398a in Arabidopsis. Here, evidence is provided for the expression of all three miR398 loci in 2-week-old Arabidopsis seedlings. By increasing the RNA quantity used for reverse transcription coupled with increased number of PCR cycles, we were able to detect the expression of the primary miR398a transcript, suggesting that miR398a is expressed at low abundance relative to miR398b and miR398c. The expression of miR398a, miR398b and miR398c loci was monitored in response to Cu²⁺ and high light stress. As shown in Figure 3B, the expression of miR398a, miR398b and miR398c precursor transcripts were down-regulated under oxidative stress conditions (Figure 3B), suggesting that the down-regulation occurs at all 3 loci.

CSD1 and CSD2 expression was simultaneously monitored in the seedlings exposed to high light, Cu²⁺, Fe³⁺ or MV (Figure 3A). The same total RNA samples were used for both CSD1 and CSD2 mRNAs and miR398 expression analysis. The CSD1 and CSD2 mRNA levels were increased in response to high light, Cu²⁺, Fe³⁺ and MV treatments (Figure 3A). Increased levels of CSD1 and CSD2 were apparent after 8 h of exposure to the stress and continued to increase with prolonged (24 h) exposure (Figure 3A).

To further correlate the stress regulation of CSD1, CSD2 and miR398, we compared their expression levels at short intervals under Cu²⁺ stress. The miR398 level was decreased within 2 h of exposure to Cu²⁺ (Figure 3C). In contrast, CSD1 and CSD2 up-regulation became apparent only at 3 h after exposure to the stress. Thus, the time course study shows that the down-regulation of miR398 preceded that of CSD1 and CSD2 mRNA up-regulation. Taken together, the above findings suggest that the lack of CSD1 and CSD2 expression in unstressed plants depends on miR398-mediated posttranscriptional regulation, and the stress induction of CSD1 and CSD2 mRNA is mediated by the down-regulation of miR398.

To gain insight into the mechanism of miR398 regulation, miR398b promoter::GUS transgenic plants were subjected to the same oxidative stress conditions (high light, Cu²⁺ and Fe³⁺) and analyzed for GUS (ß-glucuronidase) activity. Analysis of the seedlings revealed a decrease in the GUS intensity after 8 h of stress treatment, with a more pronounced decrease after 24 h of stress (Figure. 4A). A quantitative analysis of GUS activity in high light, Cu²⁺ and Fe³⁺ treatment substantiated the histochemical staining result (Figure 4B) and mirrored the Northern and RT-PCR results (Figures 3A-C). The results indicated that the down-regulation of miR398 by stress is caused by stress-induced suppression of transcription of miR398 genes.

### Oxidative stress-induced CSD1 and CSD2 expression is posttranscriptional

The results presented above clearly indicate that the stress-induced CSD1 and CSD2 mRNA is possibly caused by the suppression of miR398 expression and hence a decrease in miR398-guided CSD1 and CSD2 mRNA cleavage. However, the possibility could not be excluded that the CSD1 and CSD2 RNA levels can be transcriptionally up-regulated during these stress treatments. To determine whether there is any transcriptional regulation of the CSD1 and CSD2 genes, nuclear run-on assays were performed with 2-week-old seedlings exposed to Cu²⁺ or Fe³⁺ for 24 h. The nuclear CSD1 and CSD2 RNA levels did not differ between control and Cu²⁺ or Fe³⁺ treatments (Figure 3D). In contrast, the AtAPX1 (At1g07890) nuclear RNA level in the treated seedlings was substantially higher compared to the control and served as a positive control for the nuclear run-on assay (Figure 3D). AtAPX1 has been shown to be induced transcriptionally under oxidative stress conditions (*Fourcroy* et al., 2004). These results indicate that CSD1 and CSD2 are being transcribed in vivo at all times, with no transcriptional induction by stress. Taken together, our results show that CSD1 and CSD2 mRNA accumulation in response to oxidative stresses is a result of decreased miR398-guided posttranscriptional silencing rather than increased transcription.

### miR398 co-suppression in transgenic plants

Ectopic expression has been successfully used to analyze the role of miRNAs because each miRNA is encoded by multiple loci and this approach obviates potential problems posed by functional redundancy. Overexpression of miRNA precursors in transgenic plants can lead to increased miRNA levels and decreased target mRNA level, and such transgenic plants often phenocopy mutants with deficiencies in the target mRNA. miR398b precursor sequence was used for overexpression in transgenic plants. Despite repeated attempts, transgenic plants overexpressing miR398b were not obtained. However, plants where recovered where co-suppression had occurred (Figure 5A). We examined whether co-suppression is due to silencing of one or more of the three miR398 loci using RT-PCR designed to amplify the locus specific precursor transcripts. Very low primary transcript levels were detected for both miR398b and miR398c in co-suppression lines compared to wild-type plants (Figure 5B), suggesting that these two loci were silenced. miR398c primary transcript has extensive similarity with primary miR398b transcript, and the similarity extends beyond the predicted fold-back structure both upstream and downstream. However, primary miR398a transcript was not silenced in the cosuppression lines (Figure 5B) and this could be due to highly divergent miR398a and miR398b precursor transcript sequences outside the mature microRNA. Note that the level of miR398a transcript is much lower compared to the miR398b or miR398c transcripts, and many more PCR cycles were required to detect miR398a transcript.

To determine whether the suppression in miR398 levels affect its target gene expression, we examined the levels of CSD1 and CSD2 transcript in 2 of these lines using Northern blot analysis. CSD1 and CSD2 mRNA levels were substantially increased in the co-suppression lines compared to the wild type (Figure 5A). After Cu²⁺ or Fe³⁺ treatment, the CSD1 and CSD2 transcript levels in the co-suppression lines were similar to those in the wild type (not shown). The result confirms that miR398 can be required for silencing CSD1 and CSD2 expression in unstressed plants. In contrast, a mutated miR398 (mut-miR398b; Figure 5C) that cannot target the wild-type CSD1 and CSD2 mRNAs could be overexpressed in transgenic plants (Figure 5C). mut-miR398b differed by 5 nucleotides compared to miR398b (Figure 5c). As expected, the CSD1 and CSD2 transcript levels were unaffected in these transgenic plants (Figure 5D).

### Overexpression of a miR398-resistant form of CSD2 leads to more dramatic improvements in stress tolerance than overexpression of wild type CSD2

Chloroplasts are a particularly rich source of ROS, especially under stress conditions (Asada, 1996; Foyer et al., 1994). Efficient removal of ROS from chloroplasts is critical, because very low concentrations of ROS can inhibit photosynthesis by oxidizing the thiol-modulated enzymes in the photosynthetic carbon reduction cycle (Kaiser, 1979). Analysis of the *Arabidopsis* CSD2 knock-down (KD-SOD) mutant demonstrated an important role for CSD2 not only during high light stress but also in the absence of stress, particularly for the water-water cycle that is essential for protection of the chloroplasts under normal growth conditions (Rhizsky et al., 2003). These observations point to a critical role of CSD2 in ROS detoxification. Therefore, we focused on the functional analysis of CSD2.

Because CSD2 accumulation is affected by posttranscriptional silencing in association with miR398, we hypothesized that ectopic expression of a miR398-resistant form of CSD2 would likely result in higher accumulation of CSD2 transcript and a pronounced increase in oxidative stress tolerance. A miR398-resistant version of CSD2 construct (designated mCSD2) was generated by introducing silent mutations into the miR398 recognition site in the CSD2 ORF along with a wild-type CSD2 construct for overexpression in transgenic *Arabidopsis.* When designing the miR398-resistant mCSD2, the corresponding amino acid sequence (Figure 6A) was not altered. Both the wild-type and mCSD2 genes were overexpressed under control of the strong, constitutive super promoter (Li et al., 2001). RNA blot analysis of the resulting transgenic plants showed that overexpression of wild-type CSD2 resulted in ∼8- to 10-fold increase in transcript levels and overexpression of mCSD2 brought about a further doubling of CSD2 mRNA levels (Figure 6B and C).

To evaluate the effects of miR398-mediated CSD2 regulation on plant stress tolerance, the wild-type and transgenic plants (normal CSD2 and mCSD2) were exposed to high intensity light conditions. By visual observation, wild-type plants showed severe symptoms of loss of chlorophyll and drying of leaves, CSD2 transgenic plants showed moderate symptoms, and the mCSD2 plants showed only mild symptoms under high light stress conditions (Figure 7A). The physiological basis of high light stress tolerance was monitored by quantification of chlorophyll, anthocyanin, lipid peroxidation and photosynthetic efficiency. The total chlorophyll content was decreased in the wild type and transgenic lines exposed to high light stress, although the extent of decline was significantly lower in the transgenic plants. The decline in total chlorophyll content was the lowest in the mCSD2 transgenic lines (Figure 7B). Another indicator of stress sensitivity is the accumulation of the purple flavonoid anthocyanin in leaves. Anthocyanin levels were determined in the wild-type and transgenic plants (CSD2 and mCSD2) (Figure 7C) after 8 days of high light stress treatment. Anthocyanin levels were increased by - 20-, 10- and 3-fold in wild-type, CSD2 and mCSD2 transgenic plants, respectively (Figure 7C). To analyze the effect of high light stress on PSII activity, we measured chlorophyll fluorescence yield (Figure 7D). The maximum quantum yield of PSII photochemistry (Fv/Fm) was similar in transgenic and wild-type plants under unstressed control conditions. The differences between the wild-type and transgenic plants after 1 day of high light stress were marginal (Figure 7D). However, after 2 days and later, the decrease in quantum yield of control plants was significantly greater than in the transgenic plants. Furthermore, the extent of decline in quantum yield was less in mCSD2 than CSD2 transgenic lines (Figure 7D).

As an estimate of general lipid peroxidation, we determined the amount of malondialdehyde (MDA), a secondary end product of the oxidation of polyunsaturated fatty acids, in wild-type and transgenic plants exposed to high light (Figure 7E). Mean MDA content did not differ substantially between wild-type and transgenic plants under control conditions, but the MDA levels were elevated in wild-type and transgenic plants exposed to high light. The lipid peroxidation was greatest in wild-type plants, lower in CSD2 plants and lowest in mCSD2-overexpressing plants (Figure 7E). Thus, *Arabidopsis* plants transformed with mCSD2 showed better resistance to high light stress than those transformed with *CSD2* (Figure 7), as reflected by retention of more chlorophyll coupled with the higher PSII activity and lower levels of anthocyanin and lipid peroxidation.

To investigate whether mCSD2 transgenic plants are more tolerant than CSD2 transgenic plants in response to Cu²⁺ stress, wild-type and transgenic seeds were sown on MS-agar plates containing different concentrations of Cu²⁺ (0, 75, 100, 150 and 175 µM) and seed germination and seedling development were monitored 18 days after imbibition (Figure 8). Seed germination was significantly better in the transgenic plants compared to the wild type at 150 *µ*M Cu²⁺ (Figure 8A and 8B). Among the transgenic plants, mCSD2 showed a very high germination rate as compared to CSD2 under high Cu²⁺ stress (Figure 8B). No obvious differences were observed with respect to seedling development between wild-type and transgenic seedlings on medium with up to 100 *µ*M Cu²⁺ (data not shown), but higher Cu²⁺ concentrations adversely affected seedling development of both wild-type and transgenic lines. In the presence of 150 *µ*M Cu²⁺, wild-type seeds germinated, but their development was significantly retarded, whereas CSD2 and mCSD2 transgenic seedlings could develop (Figure 8A). Development of mCSD2 seedlings was superior as compared to CSD2 transgenic seedlings as assessed by visual observation (Figure 8A) and biomass accumulation (Figure 8C). MDA levels were elevated in both wild-type and transgenic (CSD2 and mCSD2) seedlings grown in the presence of 150 *µ*M Cu²⁺ (Figure 8D). However, the degree of lipid peroxidation was substantially lower in transgenic plants compared to wild-type plants. Furthermore, the lipid peroxidation was significantly lower in mCSD2 plants as compared to CSD2 transgenic plants (Figure 8D).

MV exacerbates superoxide and H₂O₂ production (Asada, 1996). When seeds were germinated on MS-agar medium containing different concentrations of MV, germination efficiency did not differ between wild-type and the different transgenic lines. However, seedling development was impaired in the wild type to a larger extent than in the CSD2 and mCSD2 transgenic lines (Figure 9A). Fresh weight measurements (Figure 9B) showed that 0.25 µM MV interfered with seedling development the most with wild-type plants, less so with CSD2 plants and least with mCSD2 transgenic plants.

The observation that there was a substantial increase in CSD1 and CSD2 transcripts in miR398 co-suppression lines compared to wild-type plants prompted us to evaluate their responses to oxidative stress conditions. As expected, these co-suppression lines displayed an increased tolerance to Cu²⁺ and methyl viologen stress, in terms of seedling development and lipid peroxidation rates (Figures 8 and 9).

### Feedback regulation of endogenous CSD2 gene expression in mCSD2 transgenic plants

The introduced mutations in the miR398 target sequence created an MspI restriction site in the miR398-resistant version of CSD2 (mCSD2), which allowed us to distinguish the transgene (mCSD2) mRNA levels from that of endogenous CSD2 mRNA in mCSD2 transgenic plants. To determine the endogenous CSD2 levels in mCSD2 overexpressing plants, full-length CSD2 was amplified by reverse transcription followed by PCR amplification, and the resulting PCR product was digested with MspIrestriction enzyme. Endogenous CSD2 transcript levels were substantially reduced in mCSD2 transgenic plants as compared to wild-type plants (Figure 6D and 6E). To ascertain that the decrease in endogenous CSD2 levels in mCSD2 transgenic plants is not due to increased miR398 levels, we analyzed the miR398 levels and found them unaltered in mCSD2 transgenic lines (data not shown). Therefore, the decrease in endogenous CSD2 transcript levels in mCSD2 transgenic lines indicated a possible feedback regulation of CSD2 gene transcription by CSD2 protein accumulation.

### EXAMPLES

The following examples are offered to illustrate, but not to limit the claimed invention.

### Example 1-5' RACE analysis of mRNA cleavage

Total RNA was extracted from seedlings using Trizol reagent (Invitrogen) and Poly(A)+ mRNA purified using a Poly A purification kit (Promega). RNA ligase-mediated 5' RACE was performed with the GeneRacer kit (Invitrogen). The GeneRacer RNA Oligo adapter was directly ligated to mRNA (100 ng) without calf intestinal phosphatase and tobacco acid pyrophosphatase treatment. Initial PCR was performed with the GeneRacer 5' primer and gene-specific primers for CSD1 and CSD2. Nested PCR was performed with 1 *µ*L of the initial PCR reaction, the GeneRacer 5' nested primer, and a CSD1 or CSD2 gene-specific internal primer. After the second amplification, PCR products were gel-purified, cloned and sequenced.

### Example 2 - Plant material and growth conditions

Arabidopsis thaliana Ecotype Columbia gl-1 was used as the wild type and is the genetic background for transgenic plants, except that the analysis of hen1 mutant for which the wild-type is Landsberg erecta (Ler).

### Example 3 - Constructs and generation of transgenic plants

To generate the pBIB:miR398b construct, a 300-bp fragment surrounding the miRNA sequence that includes the foldback structure of miR398b was amplified from genomic DNA with the primers indicated - forward 5' CTAGTCTAGATTTAATCAAGTTTGCAGTA CACATGTCC (SEQ ID NO: 6) and reverse 5'CGGGGTACCACTCATTGTGGGTTTC TTTACTTCCTC (SEQ ID NO: 7); XbaI and KpnI sites are underlined. The amplified fragments were digested and cloned into XbaI and KpnI sites of pBIB downstream of the super promoter. To introduce the point mutations into the miR398b precursor, PCR was performed with miR398b containing the pBIB plasmid as a template with the mutagenic primers mut forward 5'CAGCTCTCGTTTTCATAT GTGCCTAAGTCACCCCTGCTGAGCTCTTT CTCTACCGTCCATC (SEQ ID NO: 8) and mut reverse 5'AGCCGTTGATTACTCGTA TGTGCTCAAATCTACGGTGTCGAGATCCACTACCTTCATGAT (SEQ ID NO: 9). The first-round PCR products using primer pairs miR398 forward and mutated reverse and mutated forward and miR398 reverse) were gel-purified and used as template for second amplification, and the resulting product was digested and cloned into the pBIB. This fragment was sequenced to ensure that only the desired mutations were introduced.

To generate the SP:CSD2 construct, the CSD2 (At2g28190) ORF was amplified by RT-PCR with the indicated primers forward 5' CTAGTCTAGAATGGCTGCCACC AACACAATCC (SEQ ID NO: 6) and reverse 5' CGGGGTACCTTAGAGCG GCGTCAAGCCAATC (SEQ ID NO: 7): The PCR products were first cloned into pBluescript and verified by sequencing. Then, the CSD2 ORF was released by digestion with Xbal and KpnI and subcloned into pBIB. To generate an miR398-resistant version of CSD2 (mCSD2), mutagenic primers mut forward 5' GATGAGTGCCGTCATGCCGGAGATTTAGGCA ATATAAATGCCAATGCCGATGG (SEQ ID NO: 10) and mut reverse 5' GCATTGGCATTTATATTGCCTAAATCTCCG GCATGACGGCACTCATCTTCTGGAGC (SEQ ID NO: 11) were used. The first-round PCR products were purified and used as a template for the second amplification, and the resulting product was digested and cloned into the pBIB and the clone verified by sequencing.

For miR398b promoter:GUS constructs, 2.0-kb fragments upstream from the predicted fold-back structure were amplified with the forward primer 5' CCCAAGCTT TTCTAAACCT AAAGAAACCT TAG (SEQ ID NO: 12) and reverse primer 5'CCGGAATTCT CAACCCTGTCGAGATCCACTACC (SEQ ID NO: 13); HindIII and EcoRI sites are underlined. The amplified products were digested with HindIII and EcoRI and cloned into a pBI101 plasmid.

All the constructs described were electroporated into *Agrobacterium tumifaciens* GV3101, which was used to transform A. *thaliana* by the floral dip method (Clough and Bent, 1998). T3 homozygous lines were tested for all experiments presented.

### Example 4 - Stress treatments and RNA analysis

Seeds were surface-sterilized and sown on plates containing MS media with 3% sucrose and 0.6% agar. Seeds were stratified at 4°C for 2 days and then transferred to 22°C. For high light stress, plates containing 15-day-old seedlings grown under 100 *µ*mol m⁻2 s⁻¹ were transferred to 800 *µ*mol m⁻² s⁻¹. Seedlings were harvested after 8 or 24 h of high light stress. For heavy metal or methyl viologen (MV) treatments, 15-day-old seedlings were sprayed with 100 *µ*M Cu²⁺ or 100 *µ*M Fe³⁺ or 10 *µ*M MV. Seedlings were grown under a 16/8 h light/dark cycle of fluorescent light (100 *µ*mol m⁻² s⁻¹) at 22°C. Seedlings were harvested after 8 or 24 h of stress treatment. Untreated seedlings grown under same conditions served as controls.

Total RNA was extracted from 15 day-old seedlings with Trizol reagent (Invitrogen). Total RNA was separated on 1.2 % formaldehyde-MOPS agarose gels and blotted onto Hybond-N+ membranes (Amersham Biosciences). Hybridization was carried out at 65°C with PerfectHyb Plus buffer (Sigma). Probes were labeled with 32P-dCTP by use of a Ready-To-GoTM DNA Labeling Kit (Amersham Biosciences). Blots were washed twice in 2 x SSC and 0.1% SDS for 20 min at 65 °C and once in 1 x SSC 0.1% SDS.

For analysis of small RNAs, 10 *µ*g of total RNA was separated on a denaturing 15% polyacrylamide gel and transferred electrophoretically to Hybond-N+ membranes (Amersham). Hybridization and washings were performed as previously described (Sunkar and Zhu, 2004). Relative abundance was estimated by use of Typhoon and the Image Quant software.

### Example 5 - MIR398 locus-specific RT-PCR

Total RNA was extracted with Trizol reagent (Life Technologies, Carlsbad, California, United States) from 2-week-old seedlings. Contaminating DNA was removed with RNase-free DNase (RQ1-DNase; Promega, Madison, Wisconsin, United States), and reactions were performed in 25 µl using 4 *µ*g of RNA (for MIR398b and MIR398c) or 6*µ*g of RNA (M1R398a) and the Qiagen (Valencia, California, United States) One-Step RT-PCR kit. Input RNA was normalized for each reaction using actin primers. Mock RT-PCR was performed without reverse transcriptase. RT-PCR conditions for primary miR398b and miR398c transcript amplification were as follows: 50°C for 30 min, 95°C for 15 min, 35 times (94°C for 30 s, 60°C for 30 s, 72°C for 2 min), 72°C for 10 min. For miR398a amplification used essentially the same conditions except the number of PCR cycles were increased to 50. The primer pairs used for RT-PCR and predicted amplicon sizes were [forward 5' AGAAGAAGA GAAGAACAACAGGAGGTG (SEQ ID NO: 14) and reverse 5' ATTAGTAAGGT GAAAAAATGGAACAGG (SEQ ID NO: 15) (130 bp) for MIR398a and [forward 5' TAACAAGAAGATATCAATATATCATG (SEQ ID NO: 16) and reverse 5' ACCATT TGGTAAATGAGTAAAAGCCAGCC (SEQ ID NO: 17) (180 bp)] for MIR398b and [forward 5' TCGAAACTCAAACTGTAACAGTCC (SEQ ID NO: 18) and reverse 5' ATTTGGTA AATGAATAGAA GCCACG (SEQ ID NO: 19) (240 bp)] for MIR398c. Primers used for Actin2 were [forward 5' TCTTCCGCTC TTTCTTTCCA (SEQ ID NO: 20) and reverse 5'GAGAGAACAGCTTGGATGGC (SEQ ID NO: 21) (440 bp)].

### Example 6 - Nuclear run-on assay

Nuclei were isolated from 2-week-old seedlings sprayed with Cu²⁺ or Fe³⁺ (100 *µ*M) for 24 h. The nuclei isolation and in vitro transcription reactions were carried out as described (Dorweiler et al., 2000). Comparable amounts of labeled RNA were used for filter hybridization. Slot blots on nitrocellulose membrane were prepared with 100 ng of denatured purified CSD1, CSD2, AtAPX1 and tubulin fragments obtained by PCR. For comparison, 2 to 3 slots were used for each probe. Prehybridization and hybridization were carried out as described (Dorweiler et al., 2000). Following hybridization, the strips were washed for 15 min with 5X SSC and 0.1% SDS at 42oC and then with 2X SSC and 0.1% SDS for 15 min at room temperature. The strips were visualized with use of a Typhoon phosphoimager.

### Example 7 - Stress tolerance assays

For agar-plate based assays of Cu²⁺ or MV tolerance, seeds were surface-sterilized and sown on plates containing MS media with 3% sucrose and 0.6% agar. Seeds were stratified at 4°C for 3 d and then transferred to 22°C. For Cu²⁺ or MV tolerance assays, seedlings were germinated directly on Cu²⁺ (0, 100, 150, 175 *µ*M) or MV (0 and 0.25 *µ*M) containing media. Seedlings were grown under a 16/8 h light/dark cycle of fluorescent light (100 *µ*mol m⁻² s⁻¹) at 22°C for 18 days.

For pot-grown plants to test high light stress treatments, seeds were first germinated and grown on MS-agar plates for 10 days and then transferred to pots and grown at 100 *µ*mol m⁻² s⁻¹ for another 10 days. These pots were maintained in a growth chamber with continuous light (100 *µ*mol m⁻² s⁻¹) and served as controls or were exposed to continuous high light (800 *µ*mol m⁻² s⁻¹) for 8 days and photographs were taken.

### Example 8 - Histochemical detection of GUS activity

Histochemical localization of GUS activities in the transgenic seedlings or different tissues were analyzed after incubating the transgenic plants overnight at 37°C in 1 mg/mL 5-bromo-4-chloro-3-indolyl-glucuronic acid, 5 mM potassium ferricyanide, 5 mM potassium ferrocyanide, 0.03% Triton X-100, and 0.1 M sodium phosphate buffer at pH 7.0. Tissue was cleared with 70% ethanol and samples.

### Example 9 - GUS Activity Assay

GUS activity was assayed in protein extracts by a fluorescence method with 4-methylumbelliferyl glucuronide used as a substrate (Jefferson, 1987). 4-Methylumbelliferone (MU), the fluorescent product, was quantified by use of a fluorometer. Standard solutions of MU in 0.2 M Na2CO3 were used for calibration. To prepare protein extracts, the frozen tissue was ground in liquid nitrogen, extracted with buffer (50 mM sodium phosphate, pH 7.0, 1 mM EDTA, 0.1% [v/v] Triton X-100, and 10 mM 2-mercaptoethanol), and centrifuged for 10 min at 4°C in a microcentrifuge. The fluorogenic reaction was carried out in a 1-ml volume with 1 mM 4-methylumbelliferyl-β-D-glucuronide (MUG) (Duchefa Biochemie, Haarlem, The Netherlands) in the extraction buffer supplemented with a 0.1 ml aliquot of protein extract supernatants. Protein concentration was determined according to the Bio-Rad protocol provided with the protein assay kit. GUS activity was calculated as picomoles MU per minute per milligram of protein.

### Example 10 - Gene-specific RT-PCR and digestion with MspI

Total RNA was isolated from 15-day-old seedlings of mCSD2 transgenic lines and the wild type with Trizol reagent. Two *µ*g of total RNA was used for oligo dT primed first-strand cDNA synthesis in 20 *µ*l with use of Superscript II RNase H reverse transcriptase (Invitrogen). Two *µ*l of this assay was used in a 50-*µ*l PCR reaction, which contained 5 *µ*l of 10x PCR buffer, 1.5 *µ*l of 50 mM MgC12, 1 *µ*l of 10 mM dNTPs, 1 *µ*l each of gene-specific primers (10 pmol *µ*l⁻¹), and 2.5 units of Taq polymerase. The reaction (94°C, 30 sec; 55°C, 45 sec; 72°C, 60 sec) was run for 25 cycles. To monitor that equal amounts of cDNA were synthesized, a cDNA fragment of the constitutively expressed actin2 gene was amplified simultaneously in 25 cycles. The primer sequences and predicted amplicon sizes were (forward 5'- ATGGCTGCCACCAACACAATCC (SEQ ID NO: 22) and reverse 5'- TTAGAGCGG CGTCAAGCCAATC (SEQ ID NO: 23) (651 bp) for CSD2 and (forward 5'- TCTTCCGCT CTTTCTTTCCA (SEQ ID NO: 24) and reverse 5'-GAGAGAACAG CTTGGATGGC (SEQ ID NO: 25) (440 bp) for actin2. Endogenous CSD2 and miRNA-resistant (mCSD2) transcripts were distinguished by digestion with the restriction enzyme Msp1, which cuts only the mutant form. Agarose-gel separation and ethidium-bromide staining revealed the full-length CSD2 product (651 bp) and the Msp1 digestion fragments (428 bp and 223 bp). The relative expression level of endogenous CSD2 was estimated by use of Typhoon and the Image Quant software.

### Example 11 - Transient expression in Nicotiana benthamiana

For transient expression assay, the designated constructs were transformed into *Agrobacterium tumefaciens* strain 3301. Overnight cultures grown in presence of 30 *µ*M of acetosyringone were harvested by centrifugation, and cells were resuspended in 10 mM MgCl₂, 10 mM, pH 5.6 and 150 *µ*M acetosyringone to an OD 600 of 1.0. After 2-h incubation at room temperature, the *Agrobacterium* suspension was infiltrated into expanding leaves of *Nicotiana benthamiana* with use of a needleless syringe (Llave et al., 2000). Leaves were harvested 2 days after infiltration, and small RNA extraction and blotting were performed as described above.

### Example 12 - Estimation of anthocyanin

Anthocyanin levels were measured as described previously (Rabino and Mancinelli, 1986). In brief, whole leaf tissue from 3 plants per assay were weighed and then extracted with 99:1 methanol:HCl (v/v) at 4°C. The OD530 and OD657 for each sample were measured and relative anthocyanin levels determined with the equation OD530- (0.25 x OD657) x extraction volume (ml) x 1/weight of tissue sample (g) = relative units of anthocyanin/g fresh weight of tissue.

### Example 13 - Lipid peroxidation assay

The thiobarbituric acid (TBA) test, which determines MDA as an end-product, was used to analyze lipid peroxidation (Heath and Packer, 1968; Hodges et al., 1999). Briefly, 0.2 g plant material was homogenized in 4 ml of 0.1% (w/v) TCA solution on ice. The suspension was rinsed into a centrifuge tube with an additional 1 ml of TCA. The homogenate was centrifuged at 10,000 g for 5 min, and the supernatant was collected. One ml of 20% (w/v) TCA containing 0.5% (w/v) TBA was added to a 0.5-ml aliquot of the supernatant. The mixture was kept in a boiling water bath for 30 min and then quickly cooled in an ice bath. After centrifugation at 10,000 g for 10 min, the absorbance of the supernatant was measured at 532 and 600 nm. The absorbance at 600 nm was subtracted from that at 532 nm, and the MDA concentration was calculated with its extinction coefficient 155 mM 1 cm 1 (Heath and Packer, 1968; Hodges et al., 1999). No readings of note were obtained without the addition of the reactive TBA.

It is understood that the examples and embodiments described herein are for illustrative purposes only and that various modifications or changes in light thereof will be suggested to persons skilled in the art and are to be included within the spirit and purview of this application and scope of the appended claims.

While the foregoing invention has been described in some detail for purposes of clarity and understanding, it will be clear to one skilled in the art from a reading of this disclosure that various changes in form and detail can be made without departing from the true scope of the invention. For example, many of the techniques and apparatus described above can be used in various combinations.

All publications, patents, patent applications, and/or other documents cited in this application are incorporated by reference in their entirety for all purposes to the same extent as if each individual publication, patent, patent application, and/or other document were individually indicated to be incorporated by reference for all purposes.
The following numbered paragraphs (paras.) contain further statements of various aspects of the present invention:-
**1.** A polynucleotide comprising:
   a variant sequence of a first sequence: A AGG GGT TTC CTG AGA TCA CA (SEQ ID NO: 1), or of a second sequence: T GCG GGT GAC CTG GGA AAC A (SEQ ID NO: 2);
   wherein expression of a polypeptide encoded by the polynucleotide is resistant to regulation by miR398; and,
   wherein the variant encodes a first peptide sequence R G F L R S (SEQ ID NO: 3), encodes a second peptide sequence H A G D L G N (SEQ ID NO: 4) or encodes a conservative variation of the polypeptide of SEQ ID NO: 3 or SEQ ID NO: 4.
**2.** The polynucleotide of para 1, wherein
   the sequence of the SEQ ID No: 1 variant is A AGN GGN TTN CTN AGN TCN CA (SEQ ID NO: 26), or
   the sequence of the SEQ ID NO: 2 variant is N GCN GGN GAN TTN GGN AAN A (SEQ ID NO: 27) or CAN GCN GGN GAN NTN GGN AAN A (SEQ ID NO: 30);
   wherein N is any nucleotide, and at least one of the Ns represents an nucleotide different from a corresponding nucleotide of SEQ ID NO: 1 or SEQ ID NO: 2.
**3.** The polynucleotide of para 1, wherein the polynucleotide encodes a superoxide dismutase.
**4.** The polynucleotide of para **1,** wherein the variant of SEQ ID NO: 2 is CAT GCC GGA GAT TTA GGC AAT A (SEQ ID NO: 5).
**5.** A plant comprising said polynucleotide of para **1.**
**6.** A method of reducing miR398 post transcriptional regulation mRNA levels of a gene by introducing one or more mismatching nucleotides into a miR398 target sequence of the gene.
**7.** The method of para **6,** wherein the gene encodes an enzyme that is a superoxide dismutase.
**8.** The method of para **6,** wherein from 3 to 21 mismatching nucleotides are introduced.
**9.** The method of para **6,** wherein from 5 to 8 mismatching nucleotides are introduced
**10.** The method of para **6,** wherein the sequence with mismatched nucleotides comprises A AGN GGN TNN CTN AGN TCN CA (SEQ ID NO: 26), N GCN GGN GAN TTN GGN AAN A (SEQ ID NO: 27) or CAN GCN GGN GAN NTN GGN AAN A (SEQ ID NO: 30);
   wherein N is any nucleotide, and at least one of the Ns represents an nucleotide different from the corresponding nucleotide of the original sequence.
**11.** The method of para **6,** further comprising introducing the gene encoding the enzyme into a plant.
**12.** The method of para **11,** wherein the plant is selected from the group consisting of: monocots, dicots, grasses, grains, fruit plants, vegetable plants, beans, berries, rice, wheat, corn, oats, barley, alfalfa, soy beans, peanuts, apples, melons, cherries, carrots, tobacco, grapes, lettuce, onions, potatoes, cotton and tomatoes.

## Claims

1. A nucleic acid expression construct comprising a polynucleotide encoding a superoxide dismutase (SOD); the construct comprising:
i) a SOD polynucleotide comprising a variant sequence of: T GCG GGT GAC CTG GGA AAC A (SEQ ID NO: 2), wherein the variant is N GCN GGN GAN TTN GGN AAN A (SEQ ID NO: 27) or CAN GCN GGN GAN NTN GGN AAN A (SEQ ID NO: 30), wherein N is any nucleotide, and at least one of the Ns represents a nucleotide different from a corresponding nucleotide of SEQ ID NO: 2; and, wherein the variant encodes the peptide sequence H A G D L G N (SEQ ID NO: 4); and,
ii) a promoter configured to overexpress the SOD polynucleotide.

2. The nucleic acid construct of claim 1, wherein the variant of SEQ ID NO: 2 is CAT GCC GGA GAT TTA GGC AAT A (SEQ ID NO: 5).

3. The nucleic acid construct of claim **1 or 2,** wherein expression of a polypeptide encoded by the polynucleotide is resistant to regulation by miR398.

4. The nucleic acid construct of any one of claims **1-3,** wherein the promoter is a constitutive promoter.

5. The nucleic acid construct of any one of claims **1-4,** wherein the promoter is selected from the group consisting of: a cauliflower mosaic virus (CaMV) promoter, an octopine synthase promoter, a nopaline synthase promoter, a native Ti plasmid promoter, a ribulose-1,3-bisphosphate carboxylase small subunit promoter, an E8 promoter, and a phaseolin promoter.

6. The nucleic acid construct of claim 1, further comprising a variant of A AGG GGT TTC CTG AGA TCA CA (SEQ ID NO: 1), wherein the variant is A AGN GGN TTN CTN AGN TCN CA (SEQ ID NO: 26), wherein the variant encodes the peptide sequence R G F L R S (SEQ ID NO: 3).

7. A transgenic plant expressing the construct of any one of claims **1 to 6.**

8. A method of reducing miR398 post transcriptional regulation of mRNA levels of a gene encoding a superoxide dismutase (SOD), the method comprising:
introducing one or more mismatching nucleotides into a miR398 target sequence of the gene, wherein the sequence with mismatched nucleotides is N GCN GGN GAN TTN GGN AAN A (SEQ ID NO: 27) or CAN GCN GGN GAN NTN GGN AAN A (SEQ ID NO: 30); wherein N is any nucleotide, and at least one of the Ns represents an nucleotide different from the corresponding nucleotide of the original sequence; and,
overexpressing the gene in an expression construct under the control of a promoter.

9. The method of claim 8, wherein the promoter is a constitutive promoter.

10. The method of claim **8 or 9,** wherein the promoter is selected from the group consisting of: a cauliflower mosaic virus (CaMV) promoter, an octopine synthase promoter, a nopaline synthase promoter, a native Ti plasmid promoter, a ribulose-1,3-bisphosphate carboxylase small subunit promoter, an E8 promoter, and a phaseolin promoter.

11. The method of claim **8,** further comprising introducing the expression construct into a plant cell.


SEQUENCE LISTING

<110>  The Regents of the University of California

<120>  PLANT SUPEROXIDE DISMUTASE EXPRESSION RESISTANT TO MICRO-RNA
       REGULATION

<130>  LCW/FP6907836

<140>  Unknown, filed herein
<141>  2007-04-05

<150>  07774950.5
<151>  2007-04-05

<150>  PCT/US2007/008680
<151>  2007-04-05

<150>  US60/832,038
<151>  2006-07-19

<160>  31

<170>  PatentIn version 3.4

<210>  1
<211>  21
<212>  DNA
<213>  unknown

<220>
<223>  unidentified plant sequence

<400>  1
aaggggtttc ctgagatcac a                                                21


<210>  2
<211>  20
<212>  DNA
<213>  unknown

<220>
<223>  unidentified plant sequence

<400>  2
tgcgggtgac ctgggaaaca                                                  20


<210>  3
<211>  6
<212>  PRT
<213>  unknown

<220>
<223>  unidentified plant sequence

<400>  3
Arg Gly Phe Leu Arg Ser
1               5


<210>  4

<211>  7
<212>  PRT
<213>  unknown

<220>
<223>  unidentified plant sequence

<400>  4
His Ala Gly Asp Leu Gly Asn
1               5


<210>  5
<211>  22
<212>  DNA
<213>  Artificial

<220>
<223>  variant encoding SEQ ID NO:4

<400>  5
catgccggag atttaggcaa ta                                              22


<210>  6
<211>  38
<212>  DNA
<213>  Artificial

<220>
<223>  synthetic oligonucleotide primer

<400>  6
ctagtctaga tttaatcaag tttgcagtac acatgtcc                             38


<210>  7
<211>  36
<212>  DNA
<213>  Artificial

<220>
<223>  synthetic oligonucleotide primer

<400>  7
cggggtacca ctcattgtgg gtttctttac ttcctc                               36


<210>  8
<211>  61
<212>  DNA
<213>  Artificial

<220>
<223>  synthetic oligonucleotide primer

<400>  8
cagctctcgt tttcatatgt gcctaagtca cccctgctga gctctttctc taccgtccat     60

c                                                                     61


<210>  9

<211>  60
<212>  DNA
<213>  Artificial

<220>
<223>  synthetic oligonucleotide primer

<400>  9
agccgttgat tactcgtatg tgctcaaatc tacggtgtcg agatccacta ccttcatgat      60


<210>  10
<211>  53
<212>  DNA
<213>  Artificial

<220>
<223>  synthetic oligonucleotide primer

<400>  10
gatgagtgcc gtcatgccgg agatttaggc aatataaatg ccaatgccga tgg             53


<210>  11
<211>  56
<212>  DNA
<213>  Artificial

<220>
<223>  synthetic oligonucleotide primer

<400>  11
gcattggcat ttatattgcc taaatctccg gcatgacggc actcatcttc tggagc          56


<210>  12
<211>  32
<212>  DNA
<213>  Artificial

<220>
<223>  synthetic oligonucleotide primer

<400>  12
cccaagcttt tctaaaccta aagaaacctt ag                                    32


<210>  13
<211>  33
<212>  DNA
<213>  Artificial

<220>
<223>  synthetic oligonucleotide primer

<400>  13
ccggaattct caaccctgtc gagatccact acc                                   33


<210>  14
<211>  27
<212>  DNA
<213>  Artificial

<220>
<223>  synthetic oligonucleotide primer

<400>  14
agaagaagag aagaacaaca ggaggtg                                          27


<210>  15
<211>  27
<212>  DNA
<213>  Artificial

<220>
<223>  synthetic oligonucleotide primer

<400>  15
attagtaagg tgaaaaaatg gaacagg                                          27


<210>  16
<211>  26
<212>  DNA
<213>  Artificial

<220>
<223>  synthetic oligonucleotide primer

<400>  16
taacaagaag atatcaatat atcatg                                           26


<210>  17
<211>  29
<212>  DNA
<213>  Artificial

<220>
<223>  synthetic oligonucleotide primer

<400>  17
accatttggt aaatgagtaa aagccagcc                                        29


<210>  18
<211>  24
<212>  DNA
<213>  Artificial

<220>
<223>  synthetic oligonucleotide primer

<400>  18
tcgaaactca aactgtaaca gtcc                                             24


<210>  19
<211>  25
<212>  DNA
<213>  Artificial

<220>
<223>  synthetic oligonucleotide primer

<400>  19
atttggtaaa tgaatagaag ccacg                                              25


<210>  20
<211>  20
<212>  DNA
<213>  Artificial

<220>
<223>  synthetic oligonucleotide primer

<400>  20
tcttccgctc tttctttcca                                                    20


<210>  21
<211>  20
<212>  DNA
<213>  Artificial

<220>
<223>  synthetic oligonucleotide primer

<400>  21
gagagaacag cttggatggc                                                    20


<210>  22
<211>  22
<212>  DNA
<213>  Artificial

<220>
<223>  synthetic oligonucleotide primer

<400>  22
atggctgcca ccaacacaat cc                                                 22


<210>  23
<211>  22
<212>  DNA
<213>  Artificial

<220>
<223>  synthetic oligonucleotide primer

<400>  23
ttagagcggc gtcaagccaa tc                                                 22


<210>  24
<211>  20
<212>  DNA
<213>  Artificial

<220>
<223>  synthetic oligonucleotide primer

<400>  24
tcttccgctc tttctttcca                                                    20

<210>  25
<211>  20
<212>  DNA
<213>  Artificial

<220>
<223>  synthetic oligonucleotide primer

<400>  25
gagagaacag cttggatggc                                                    20


<210>  26
<211>  21
<212>  DNA
<213>  Artificial

<220>
<223>  variant of SEQ ID NO:1


<220>
<221>  misc_feature
<222>  (4)..(4)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (7)..(7)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (10)..(10)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (13)..(13)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (16)..(16)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (19)..(19)
<223>  N is any nucleotide, where at least one of the Ns in this SEQ ID
       NO represents a nucleotide different from the corresponding

nucleotide of SEQ ID NO: 1

<400> 26
aagnggnttn ctnagntcnc a 21

<210> 27
<211> 20
<212> DNA
<213> Artificial

<220>
<223> variant of SEQ ID NO:2

<220>
<221> misc_feature
<222> (1)..(1)
<223> N is any nucleotide, where at least one of the Ns in this SEQ ID
NO represents a nucleotide different from the corresponding
nucleotide of SEQ ID NO: 2

<220>
<221> misc_feature
<222> (4)..(4)
<223> N is any nucleotide, where at least one of the Ns in this SEQ ID
NO represents a nucleotide different from the corresponding
nucleotide of SEQ ID NO: 2

<220>
<221> misc_feature
<222> (7)..(7)
<223> N is any nucleotide, where at least one of the Ns in this SEQ ID
NO represents a nucleotide different from the corresponding
nucleotide of SEQ ID NO: 2

<220>
<221> misc_feature
<222> (10)..(10)
<223> N is any nucleotide, where at least one of the Ns in this SEQ ID
NO represents a nucleotide different from the corresponding
nucleotide of SEQ ID NO: 2

<220>
<221> misc_feature
<222> (13)..(13)
<223> N is any nucleotide, where at least one of the Ns in this SEQ ID
NO represents a nucleotide different from the corresponding
nucleotide of SEQ ID NO: 2

<220>
<221> misc_feature
<222> (16)..(16)
<223> N is any nucleotide, where at least one of the Ns in this SEQ ID
NO represents a nucleotide different from the corresponding
nucleotide of SEQ ID NO: 2

<220>
<221> misc_feature
<222> (19)..(19)
<223> N is any nucleotide, where at least one of the Ns in this SEQ ID
NO represents a nucleotide different from the corresponding
nucleotide of SEQ ID NO: 2

<400>  27
ngcnggngan ttnggnaana                                                  20


<210>  28
<211>  20
<212>  DNA
<213>  Nicotiana plumbaginifolia

<400>  28
aggggttccc tgagatcaca                                                  20


<210>  29
<211>  20
<212>  DNA
<213>  Artificial

<220>
<223>  variant of SEQ ID NO:1


<220>
<221>  misc_feature
<222>  (3)..(3)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (6)..(6)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (8)..(9)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (12)..(12)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (15)..(15)
<223>  N is any nucleotide, where at least one of the Ns in this SEQ ID
       NO represents a nucleotide different from the corresponding
       nucleotide of SEQ ID NO: 1

<220>
<221>  misc_feature
<222>  (18)..(18)
<223>  N is any nucleotide, where at least one of the Ns in this SEQ ID
       NO represents a nucleotide different from the corresponding

nucleotide of SEQ ID NO: 1

<400> 29
agnggntnnc tnagntcnca 20


<210> 30
<211> 22
<212> DNA
<213> Artificial

<220>
<223> variant of SEQ ID NO:2


<220>
<221> misc_feature
<222> (3)..(3)
<223> N is any nucleotide, where at least one of the Ns in this SEQ ID
NO represents a nucleotide different from the corresponding
nucleotide of SEQ ID NO: 2

<220>
<221> misc_feature
<222> (6)..(6)
<223> N is any nucleotide, where at least one of the Ns in this SEQ ID
NO represents a nucleotide different from the corresponding
nucleotide of SEQ ID NO: 2

<220>
<221> misc_feature
<222> (9)..(9)
<223> N is any nucleotide, where at least one of the Ns in this SEQ ID
NO represents a nucleotide different from the corresponding
nucleotide of SEQ ID NO: 2

<220>
<221> misc_feature
<222> (12)..(13)
<223> N is any nucleotide, where at least one of the Ns in this SEQ ID
NO represents a nucleotide different from the corresponding
nucleotide of SEQ ID NO: 2

<220>
<221> misc_feature
<222> (15)..(15)
<223> N is any nucleotide, where at least one of the Ns in this SEQ ID
NO represents a nucleotide different from the corresponding
nucleotide of SEQ ID NO: 2

<220>
<221> misc_feature
<222> (18)..(18)
<223> N is any nucleotide, where at least one of the Ns in this SEQ ID
NO represents a nucleotide different from the corresponding
nucleotide of SEQ ID NO: 2

<220>
<221> misc_feature
<222> (21)..(21)
<223> N is any nucleotide, where at least one of the Ns in this SEQ ID
NO represents a nucleotide different from the corresponding
nucleotide of SEQ ID NO: 2

<400>  30
cangcnggng anntnggnaa na                                                22

<210>  31
<211>  20
<212>  DNA
<213>  Arachis hypogaea

<400>  31
agggggttccc tgagatcaca                                                  20

3/10

2/10